(19) Europäisches Patentamt
European Patent Office
Office européen des brevets

(11) Veröffentlichungsnummer: **0 264 021**
**A2**

(12) # EUROPÄISCHE PATENTANMELDUNG

(21) Anmeldenummer: **87114215.4**

(22) Anmeldetag: **29.09.87**

(51) Int. Cl.4: **C07D 251/16**, C07D 251/22, C07D 251/46, C07D 251/18

(30) Priorität: **14.10.86 DE 3634928**

(43) Veröffentlichungstag der Anmeldung:
**20.04.88 Patentblatt 88/16**

(84) Benannte Vertragsstaaten:
**CH DE FR GB LI**

(71) Anmelder: **BAYER AG**
**Konzernverwaltung RP Patentabteilung**
**D-5090 Leverkusen 1 Bayerwerk(DE)**

(72) Erfinder: **Fest, Christa, Dr.**
**Im Johannistal 20**
**D-5600 Wuppertal 1(DE)**
Erfinder: **Riebel, Hans-Jochem, Dr.**
**In der Beek 92**
**D-5600 Wuppertal 1(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Robert Harry, Dr.**
**Unterdorfstrasse 6A**
**D-4000 Düsseldorf 31(DE)**

(54) **0-Aryl-N'-(triazin-2-yl)-isoharnstoffe.**

(57) Die Erfindung betrifft neue O-Aryl-N'-(triazin-2-yl)isoharnstoffe der allgemeinen der Formel (I)

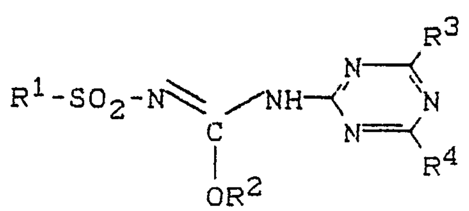

(I)

(worin $R^1$, $R^2$, $R^3$ und $R^4$ die in der Beschreibung angegebenen Bedeutungen haben), ein Verfahren zur Ihrer Herstellung und ihre Verwendung als Herbizide.

EP 0 264 021 A2

## O-Aryl-N'-(triazin-2-yl)-isoharnstoffe

Die Erfindung betrifft neue O-Aryl-N'-(triazin-2-yl)-isoharnstoffe, ein Verfahren zu ihrer Herstellung sowie ihre Verwendung als Herbizide.

Es ist bekannt, daß bestimmte Isoharnstoffe, wie z.B. N'-(2-Chlor-benzolsulfonyl)-N''-(4,6-dimethyl-pyrimidin-2-yl)-O-(4-chlorphenyl)-isoharnstoff und N'-(2-Chlor-benzolsulfonyl)-N''-(4,6-dimethyl-1,3,5-triazin-2-yl)-O-phenyl-isoharnstoff, herbizide Eigenschaften besitzten. Die Wirkung dieser Verbindungen ist jedoch nicht immer ganz befriedigend (vgl. z. B. CH-PS 646 957 und EP-A 173 957).

Es wurden nun neue O-Aryl-N'-(triazin-2-yl)-isoharnstoffe der allgemeinen Formel (I)

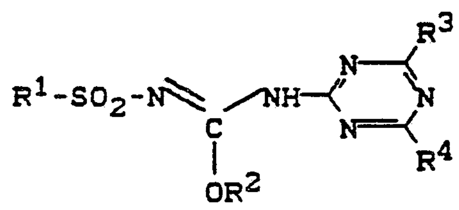

(I)

in welcher

$R^1$ für den Rest

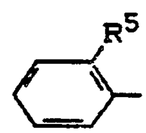

steht, worin

$R^5$ für Trifluormethoxy, Difluormethoxy, Phenyl, Phenoxy, Trifluormethylthio, Difluormethylthio, Di-($C_1$-$C_4$-alkyl)-aminocarbonyl, $C_1$-$C_4$-Alkylsulfonyl, $C_1$-$C_4$-Alkoxyaminosulfonyl, N-($C_1$-$C_4$-Alkoxy)-N-($C_1$-$C_4$-alkyl)-amino-sulfonyl, $C_1$-$C_4$-Alkylaminosulfonyl oder Di-($C_1$-$C_4$-alkyl)-aminosulfonyl steht,

worin weiter

$R^1$ für den Rest

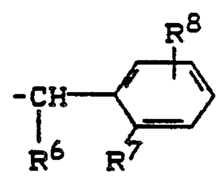

steht, worin

$R^6$ für Wasserstoff oder $C_1$-$C_4$-Alkyl steht,

$R^7$ und $R^8$ gleich oder verschieden sind und für Was serstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxycarbonyl, $C_1$-$C_4$-Alkylsulfonyl oder Di-($C_1$-$C_4$-alkyl)-aminosulfonyl stehen;

worin weiter

$R^2$ für gegebenenfalls substituiertes Aryl steht,

$R^3$ für Wasserstoff, Halogen, Hydroxy, $C_1$-$C_6$-Alkylamino, Di-($C_1$-$C_6$-alkyl)-amino oder für gegebenenfalls substituiertes $C_1$-$C_6$-Alkyl, $C_1$-$C_6$-Alkoxy und $C_1$-$C_6$-Alkylthio steht und

$R^4$ für Wasserstoff, Halogen, Hydroxy, für gegebenenfalls substituiertes $C_1$-$C_6$-Alkyl, $C_1$-$C_6$-Alkoxy und $C_1$-$C_6$-Alkylthio, für $C_1$-$C_6$-Alkylamino oder Di-($C_1$-$C_6$-alkyl)-amino steht,

gefunden.

Man erhält die neuen O-Aryl-N'-(triazin-2-yl)-isoharnstoffe der allgemeinen Formel (I), wenn man Iminokohlensäureester der Formel (II)

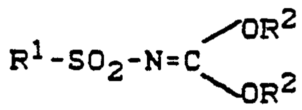 (II)

in welcher
$R^1$ und $R^2$ die oben angegebenen Bedeutungen haben,
mit 2-Amino-triazinen der Formel (III)

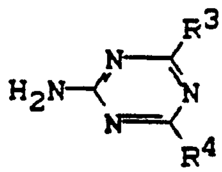 (III)

in welcher
$R^3$ und $R^4$ die oben angegebenen Bedeutungen haben,
in Gegenwart von Säureakzeptoren und gegebenenfalls in Gegenwart von Verdünnungsmitteln umsetzt.

Die neuen O-Aryl-N'-(triazin-2-yl)-isoharnstoffe der Formel (I) zeichnen sich durch starke herbizide Wirksamkeit aus. Überraschenderweise zeigen die neuen Verbin dungen der Formel (I) eine erheblich bessere herbizide Wirkung als vorbekannte Isoharnstoffe gleicher Wirkungsrichtung.

Gegenstand der Erfindung sind vorzugsweise Verbindungen der Formel (I), in welcher
$R^1$ für den Rest

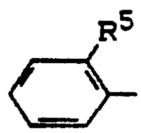

steht, worin
$R^5$ für Trifluormethoxy, Difluormethoxy, Phenyl, Phenoxy, Trifluormethylthio, Difluormethylthio, Di-($C_1$-$C_4$-alkyl)-aminocarbonyl, $C_1$-$C_3$-Alkylsulfonyl, $C_1$-$C_4$-Alkoxyaminosulfonyl, N-($C_1$-$C_2$-Alkoxy)-N-($C_1$-$C_2$-alkyl)-aminosulfonyl, $C_1$-$C_2$-Alkylaminosulfonyl oder Di-($C_1$-$C_2$-alkyl)-aminosulfonyl steht,
worin weiter
$R^1$ für den Rest

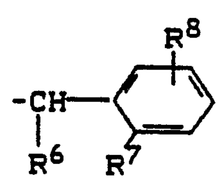

steht, worin
$R^6$ für Wasserstoff oder $C_1$-$C_4$-Alkyl steht,
$R^7$ und $R^8$ gleich oder verschieden sind und für Wasserstoff, Fluor, Chlor, Brom, Cyano, Methyl, Methoxy, Trifluormethyl, Trifluormethoxy, Difluormethoxy, Carboxy, Methoxycarbonyl, Ethoxycarbonyl, Methylsulfonyl, Ethylsulfonyl, Dimethylaminosulfonyl und Diethylaminosulfonyl stehen;
worin weiter
$R^2$ für einen Phenylrest steht, welcher gegebenenfalls substituiert ist durch einen oder mehrere Reste aus der Reihe
Halogen [wie insbesondere Fluor, Chlor, Brom und Iod], Cyano, Nitro, Hydroxy, Carboxy, $C_1$-$C_6$-Alkyl - [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxy-carbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welche gegebenen-

falls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonyl-amino, $C_1$-$C_4$-Alkoxy-carbonylamino, (Di)-$C_1$-$C_4$-Alkylamino-carbonyl-amino, Formyl, $C_1$-$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkyl-amino-carbonyloxy und Di-($C_1$-$C_4$-alkyl)-amino-carbonyloxy, oder welcher gegebenenfalls durch eine Alkylenkette [welche gegebenenfalls verzweigt und/oder durch ein oder mehrere Sauerstoffatome unterbrochen ist] oder ei nen Benzorest [welcher gegebenenfalls durch Fluor, Chlor, Brom, Cyano, Nitro, Methyl und/oder Trifluormethyl substituiert ist] anelliert ist; worin weiter

$R^3$ für Wasserstoff, Fluor, Chlor, Brom, Hydroxy, $C_1$-$C_4$-Alkylamino, Di-($C_1$-$C_4$-alkyl)-amino, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist] steht und

$R^4$ für Wasserstoff, Fluor, Chlor, Brom, Hydroxy, $C_1$-$C_4$-Alkyl [welches gegebenenfalls durch Fluor und/oder Chlor substituierte ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], $C_1$-$C_4$-Alkyl-amino oder Di-($C_1$-$C_4$-alkyl)-amino steht.

Besonders bevorzugt sind diejenigen Verbindungen der Formel (I), in welcher

$R^1$ für den Rest

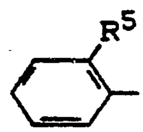

steht, worin

$R^5$ für Difluormethoxy, Trifluormethoxy, Difluormethylthio, Trifluormethylthio, Dimethylaminocarbonyl, Diethylaminocarbo nyl, Di-n-propylaminocarbonyl, Methylsulfonyl, Ethylsulfonyl, n-Propylsulfonyl, i-Propylsulfonyl, Dimethylaminosulfonyl, Diethylaminosulfonyl, Methoxyaminosulfonyl, N-Methoxy-N-methylaminosulfonyl, Phenyl oder für Phenoxy, steht und

$R^2$ für einen Phenylrest steht, welcher gegebenenfalls substituiert ist durch einen oder zwei Reste aus der Reihe

Fluor, Chlor, Brom, Iod, Cyano, Nitro, Hydroxy, Carboxy, $C_1$-$C_3$-Alkoxy-carbonyl, $C_1$-$C_4$-Alkyl, Trifluormethyl, Hydroxymethyl, Methoxycarbonylmethyl, Phenyl-$C_1$-$C_3$-alkyl, Cyclohexyl, $C_1$-$C_3$-Alkoxy, Trifluormethoxy, $C_1$-$C_3$-Alkylthio, Trifluormethylthio, Dimethylamino, Amino, Acetylamino, Methylaminocarbonyl, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pyridoxy [welches gegebenenfalls durch Chlor und/oder Trifluormethyl substituiert ist], oder welcher gegebenenfalls benzanelliert ist; worin weiter

$R^3$ für Fluor, Chlor, Methyl, Ethyl, Methoxy, Ethoxy, Methylthio, Ethylthio, Methylamino, Ethylamino, Dimethylamino oder Diethylamino steht und

$R^4$ für Fluor, Chlor, Methyl, Ethyl, Methoxy, Ethoxy, Methylthio, Ethylthio, Methylamino, Ethylamino, Dimethylamino oder Diethylamino steht.

Verwendet man beispielsweise für das erfindungsgemäße Verfahren N-(2-Trifluormethoxy-benzolsulfonyl)-iminokohlensäure-O,O-diphenylester und 2-Amino-4-methoxy-6-methyl-1,3,5-triazin als Ausgangsstoffe, so kann der Reaktionsablauf durch das folgende Formelschema wiedergegeben werden:

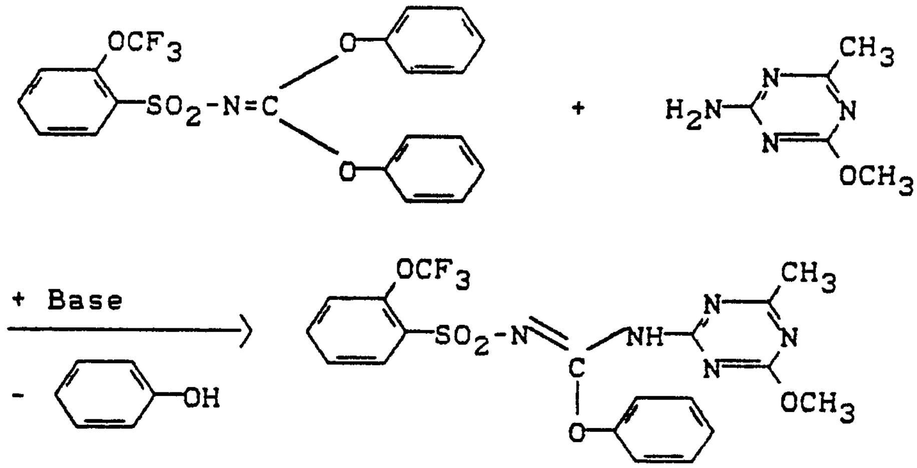


Die beim erfindungsgemäßen Verfahren als Ausgangsstoffe zu verwendenden Iminokohlensäureester sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) vorzugsweise für diese Substituenten genannt wurden.

Die Verbindungen der Formel (II) sind neu und Gegenstand einer eigenen, hiermit gleichzeitig eingreichten und nicht zum vorveröffentlichten Stand der Technik gehörenden Patentanmeldung (vgl. die deutsche Anmeldung P 36 34 926.7).

Man erhält die Verbindungen der Formel (II), wenn man Iminokohlensäuredichloride der Formel (IV)

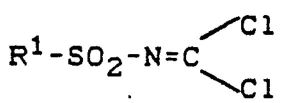 (IV)

in welcher
$R^1$ die oben angegebenen Bedeutungen hat,
mit Verbindungen der Formel (V)
$R^2$-OM (V)
in welcher
$R^2$ die oben angegebenen Bedeutungen hat und
M für Wasserstoff oder ein Alkalimetallatom steht,
gegebenenfalls in Gegenwart von Säureakzeptoren, wie z. B. Natrium-oder Kaliumhydroxid, und gegebenenfalls in Gegenwart von inerten Verdünnungsmitteln, wie z. B. Aceton, Methylethylketon oder Acetonitril, bei Temperaturen zwischen 10 °C und 100 °C umsetzt.

Die Iminokohlensäuredichloride der Formel (IV) sind neu. Sie sind ebenfalls Gegenstand einer eigenen, hiermit gleichzeitig eingereichten und nicht zum vorveröffentlichten Stand der Technik gehörenden Patentanmeldung (vgl. die deutsche Anmeldung P 36 34 926.7).

Man erhält die neuen Verbindungen der Formel (IV), wenn man Iminodithiokohlensäuredimethylester der Formel (VI)

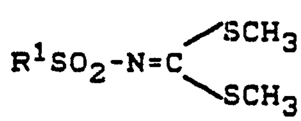 (VI)

in welcher
$R^1$ die oben angegebenen Bedeutungen hat,
in Gegenwart von inerten Verdünnungsmitteln, wie z. B. Tetrachlorkohlenstoff, bei Temperaturen zwischen 0 °C und 25 °C mit Chlorierungsmitteln, wie z. B. Sulfurylchlorid oder Chlor umsetzt.

Die Iminodithiokohlensäuredimethylester der Formel (VI) sind bekannt und/oder lassen sich nach bekannten Methoden herstellen (vergl. z. B. EP-A 121 082, EP-A 151 554 und EP-A 173 957).

Die weiterhin als Ausgangsstoffe für die Herstellung der Verbindungen der Formel (II) einzusetzenden Verbindungen der Formel (V) sind allgemein bekannte Verbindungen der organischen Chemie.

Als Beispiele für die Iminokohlensäureester der Formel (II) seien genannt:

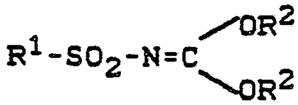 (II)

**Tabelle 1**

| $R^1$ | $R^2$ |
|---|---|
| 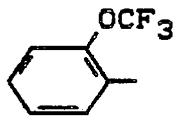 | 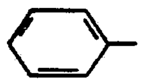 |
| 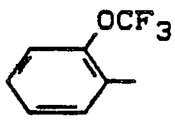 | 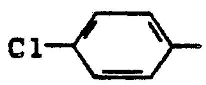 |
| 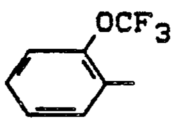 | 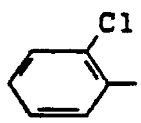 |
| 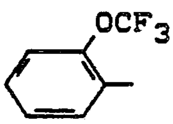 | 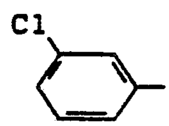 |
| 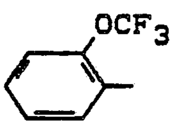 | 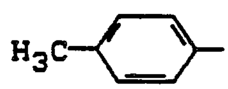 |
| 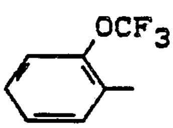 | 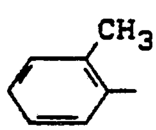 |
| 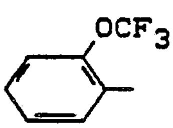 | 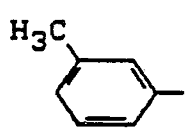 |
| 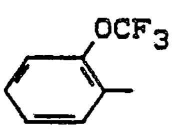 | 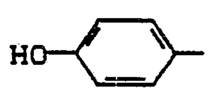 |

Tabelle 1 - Fortsetzung

| $R^1$ | $R^2$ |
| --- | --- |
| 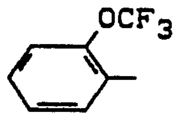 | 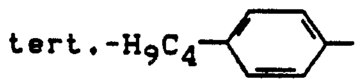 |
| 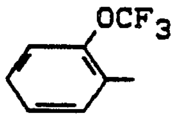 | 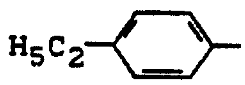 |
| 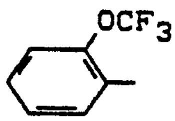 | 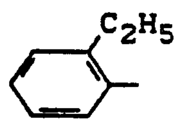 |
| 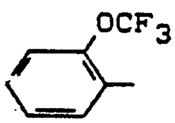 | 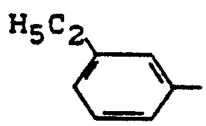 |
| 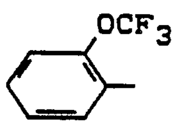 | 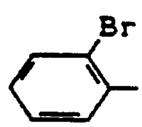 |
| 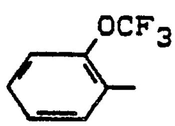 | 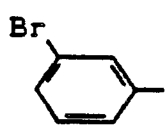 |
| 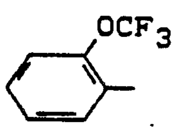 | 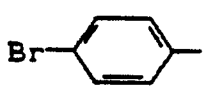 |
| 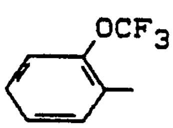 | 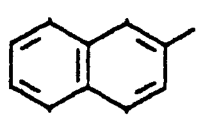 |

Tabelle 1 - Fortsetzung

| $R^1$ | $R^2$ |
|---|---|
| 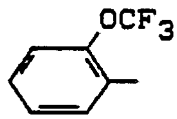 | 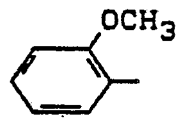 |
| 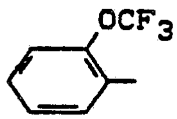 | 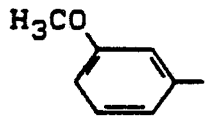 |
| 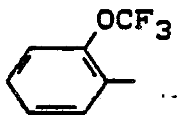 | 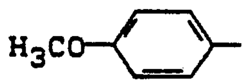 |
| 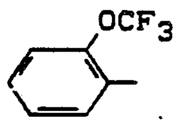 | 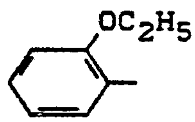 |
| 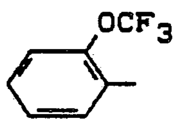 | 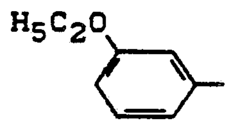 |
| 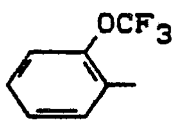 | 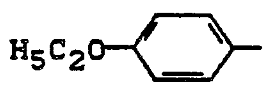 |
| 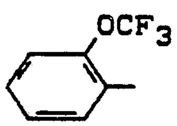 | 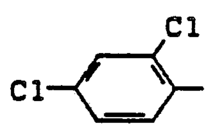 |
| 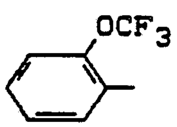 | 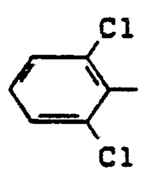 |

Tabelle 1 - Fortsetzung

| $R^1$ | $R^2$ |
|---|---|
| 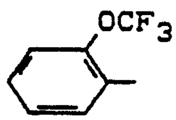 | 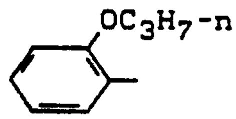 |
| 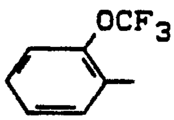 | 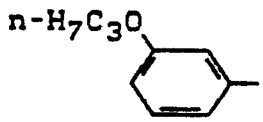 |
| 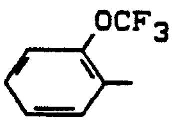 | 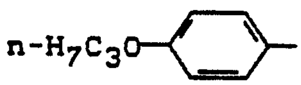 |
| 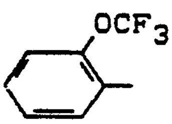 | 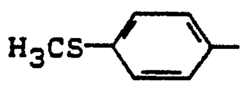 |
| 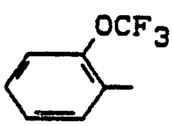 | 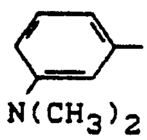 |
| 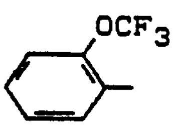 | 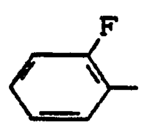 |
| 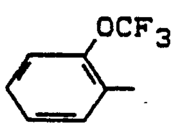 | 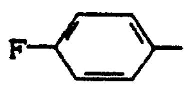 |
| 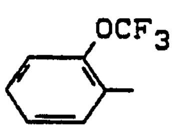 | 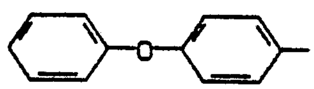 |

Tabelle 1 - Fortsetzung

| $R^1$ | $R^2$ |
|---|---|
| 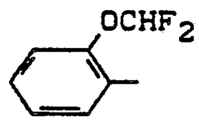 | 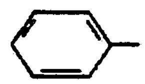 |
| 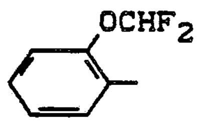 | 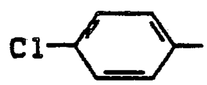 |
| 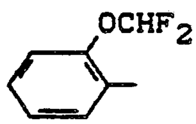 | 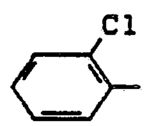 |
| 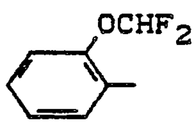 | 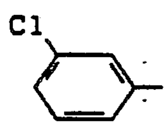 |
| 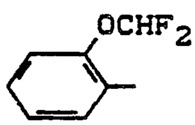 | 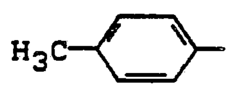 |
| 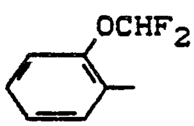 | 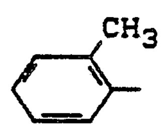 |
| 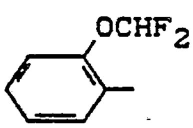 | 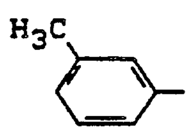 |
| 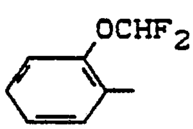 | 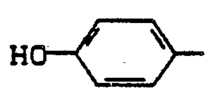 |

<u>Tabelle 1</u> - Fortsetzung

| $R^1$ | $R^2$ |
|---|---|
| 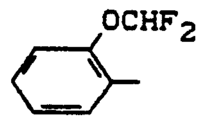 | 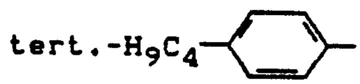 |
| 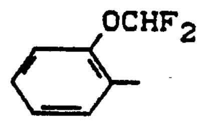 | 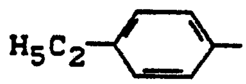 |
| 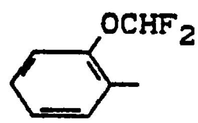 | 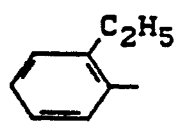 |
| 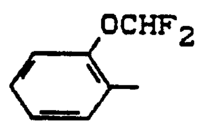 | 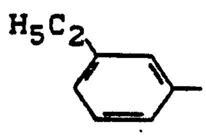 |
| 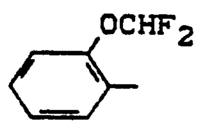 | 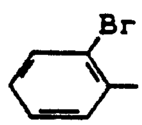 |
| 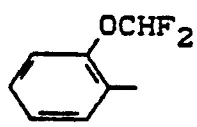 | 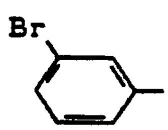 |
| 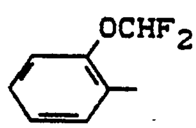 | 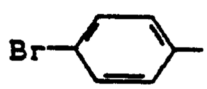 |
| 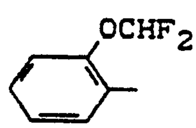 | 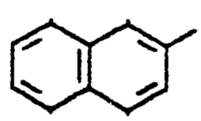 |

Tabelle 1 - Fortsetzung

| $R^1$ | $R^2$ |
|---|---|
| 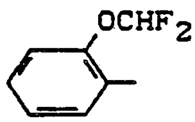 | 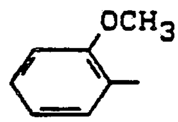 |
| 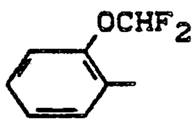 | 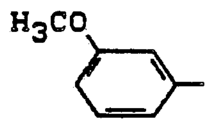 |
| 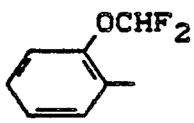 | 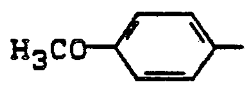 |
| 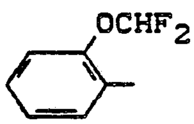 | 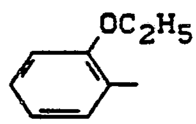 |
| 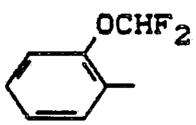 | 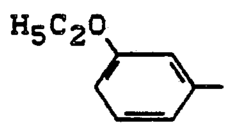 |
| 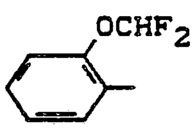 | 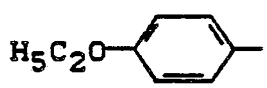 |
| 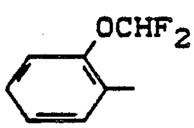 | 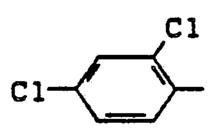 |
| 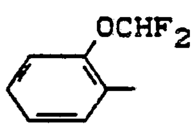 | 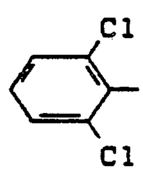 |

Tabelle 1 - Fortsetzung

| $R^1$ | $R^2$ |
|---|---|
| 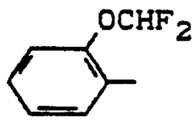 | 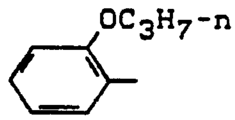 |
| 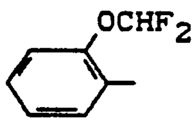 | 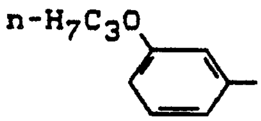 |
| 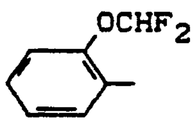 | 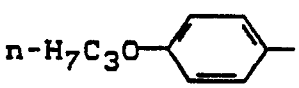 |
| 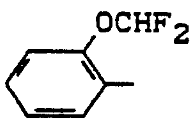 | 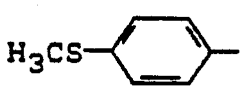 |
| 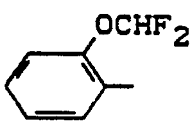 | 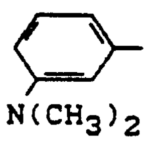 |
| 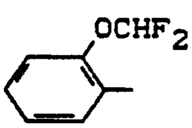 | 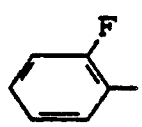 |
| 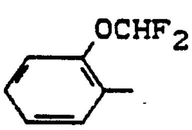 | 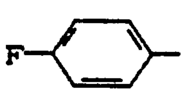 |
| 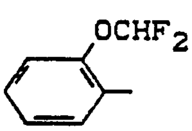 | 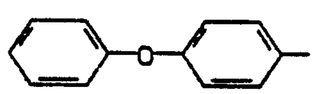 |

<u>Tabelle 1</u> - Fortsetzung

| $R^1$ | $R^2$ |
|---|---|
| 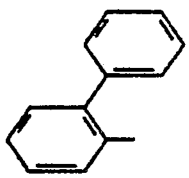 | 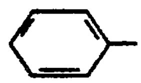 |
| 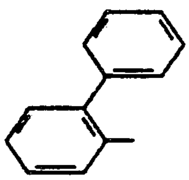 | 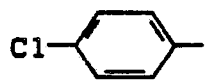 |
| 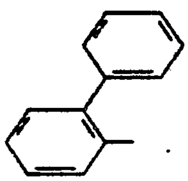 | 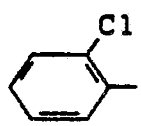 |
| 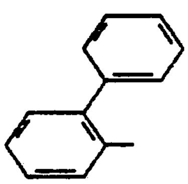 | 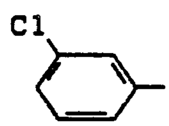 |
| 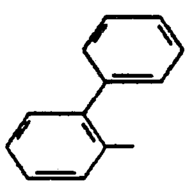 | 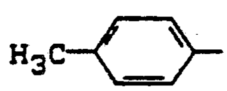 |
| 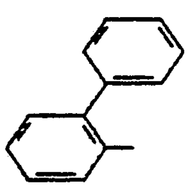 | 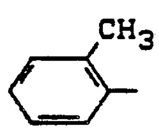 |

Tabelle 1 - Fortsetzung

| $R^1$ | $R^2$ |
|---|---|
| 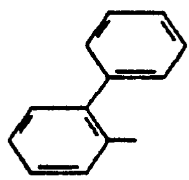 | 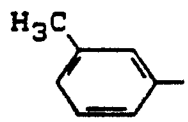 |
| 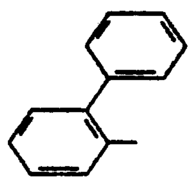 | 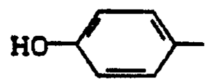 |
| 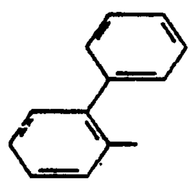 | 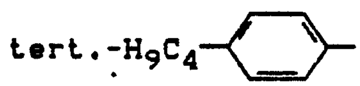 |
| 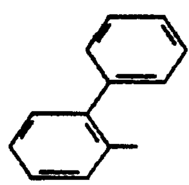 | 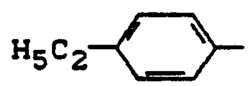 |
| 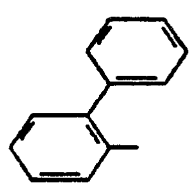 | 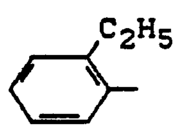 |
| 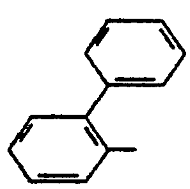 | 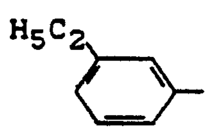 |

Tabelle 1 - Fortsetzung

| $R^1$ | $R^2$ |
|---|---|
| 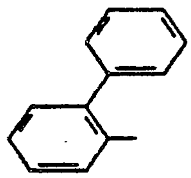 | 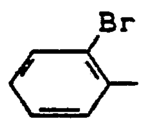 |
| 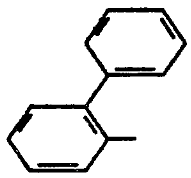 | 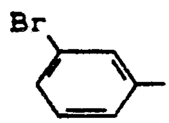 |
| 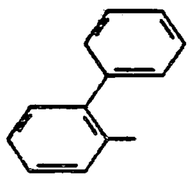 | 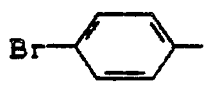 |
| 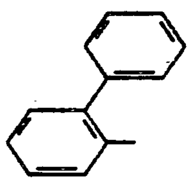 | 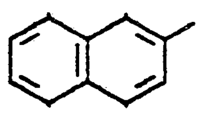 |
| 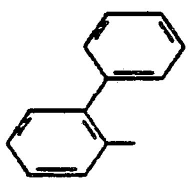 | 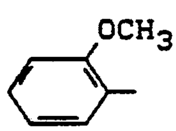 |
| 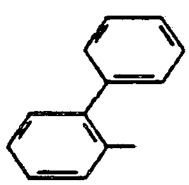 | 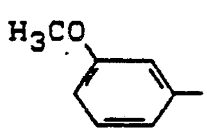 |

Tabelle 1 - Fortsetzung

| $R^1$ | $R^2$ |
|---|---|
| 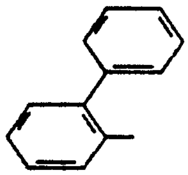 | 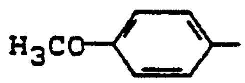 |
| 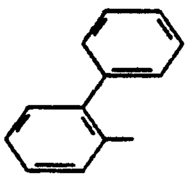 | 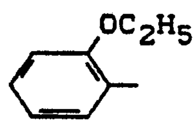 |
| 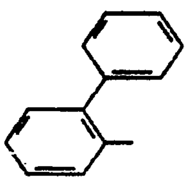 | 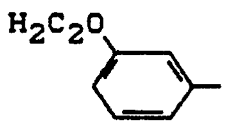 |
| 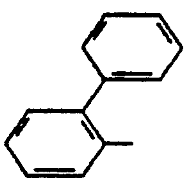 | 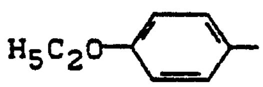 |
| 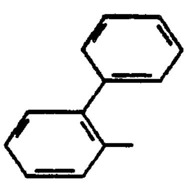 | 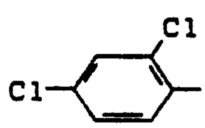 |
| 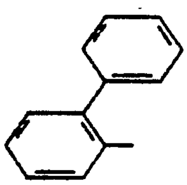 | 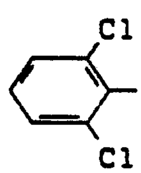 |

Tabelle 1 - Fortsetzung

| $R^1$ | $R^2$ |
|---|---|
| 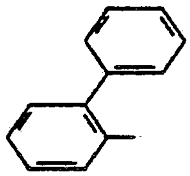 | 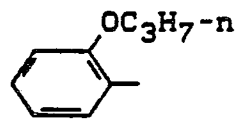 |
| 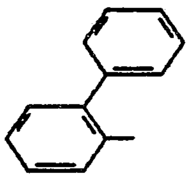 | 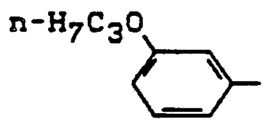 |
| 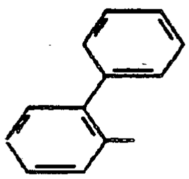 | 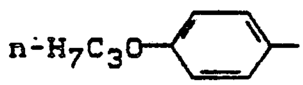 |
| 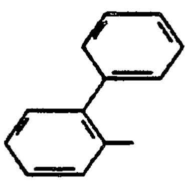 | 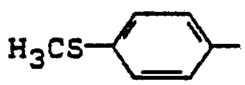 |
| 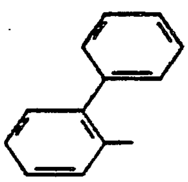 | 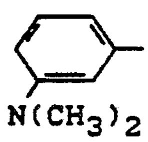 |
| 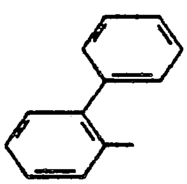 | 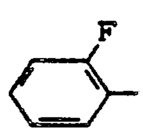 |

Tabelle 1 - Fortsetzung

| $R^1$ | $R^2$ |
|---|---|
| 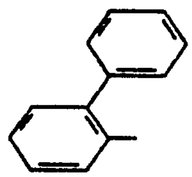 | 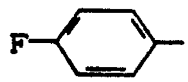 |
| 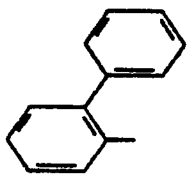 | 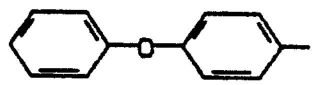 |
| 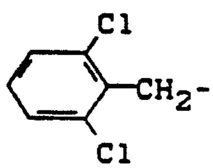 | 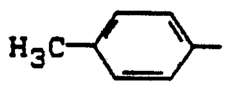 |
| 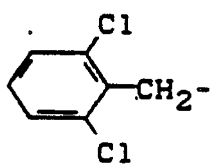 | 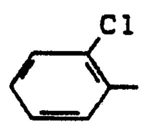 |
| 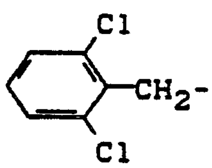 | 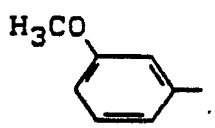 |
| 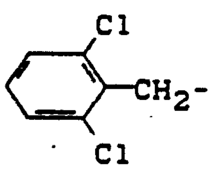 | 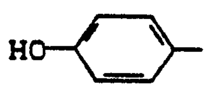 |
| 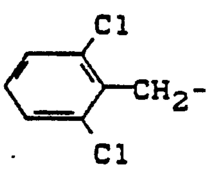 | 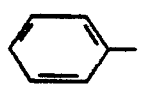 |

Tabelle 1 - Fortsetzung

| $R^1$ | $R^2$ |
|---|---|
| 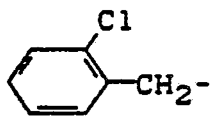 | 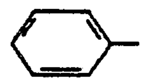 |
| 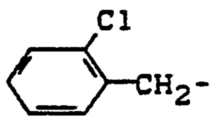 | 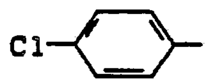 |
| 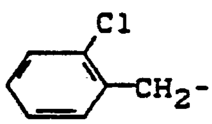 | 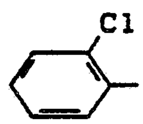 |
| 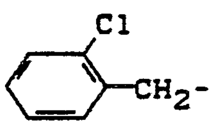 | 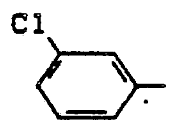 |
| 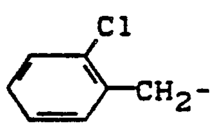 | 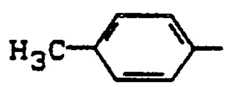 |
| 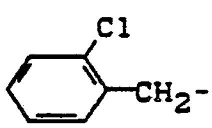 | 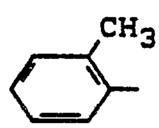 |
| 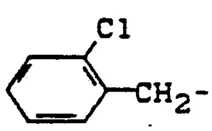 | 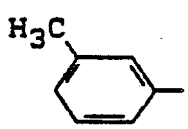 |
| 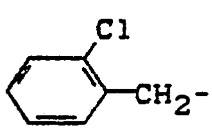 | 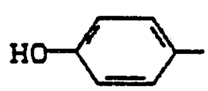 |

<u>Tabelle 1</u> - Fortsetzung

| $R^1$ | $R^2$ |
|---|---|
| 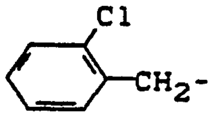 | 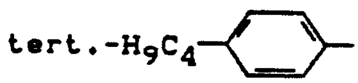 |
| 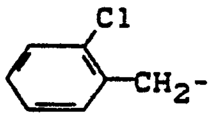 | 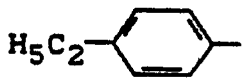 |
| 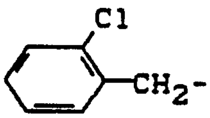 | 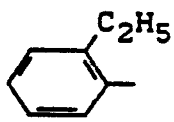 |
| 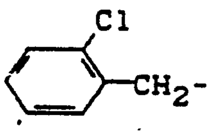 | 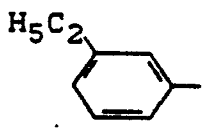 |
| 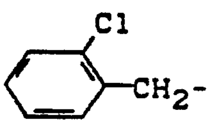 | 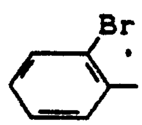 |
| 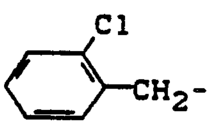 | 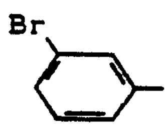 |
| 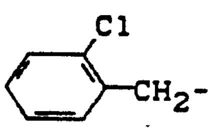 | 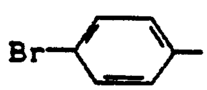 |
| 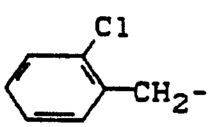 | 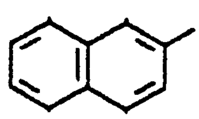 |

Tabelle 1 - Fortsetzung

| $R^1$ | $R^2$ |
|---|---|
| 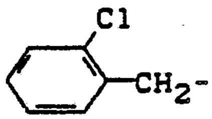 | 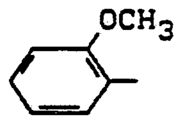 |
| 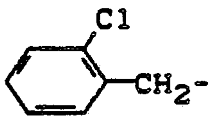 | 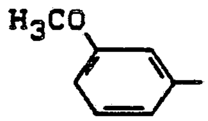 |
| 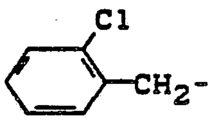 | 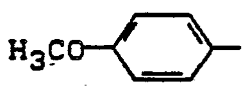 |
| 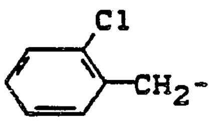 | 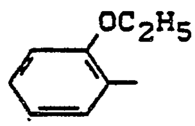 |
| 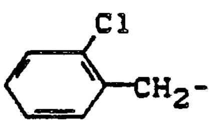 | 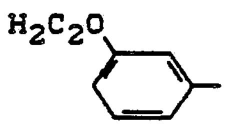 |
| 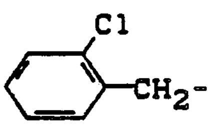 | 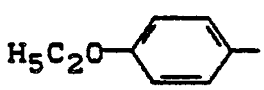 |
| 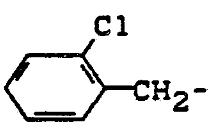 | 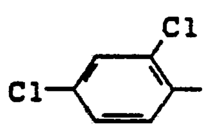 |
| 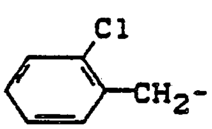 | 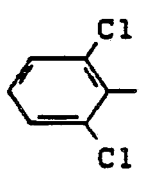 |

Tabelle 1 - Fortsetzung

| $R^1$ | $R^2$ |
|---|---|
| 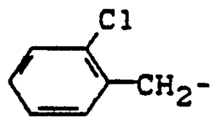 | 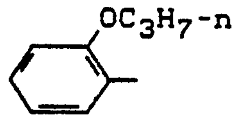 |
| 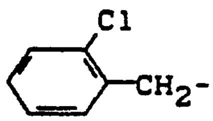 | 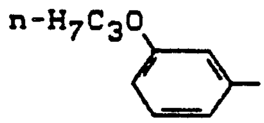 |
| 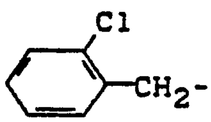 | 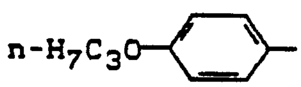 |
| 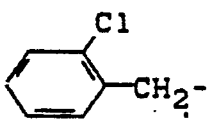 | 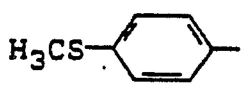 |
| 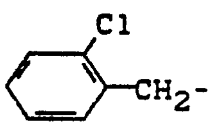 | 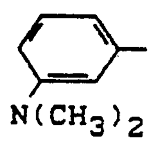 |
| 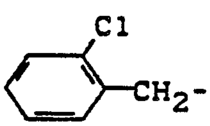 | 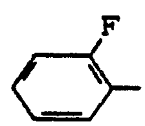 |
| 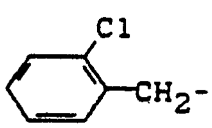 | 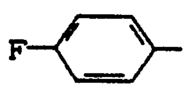 |
| 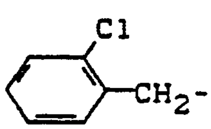 | 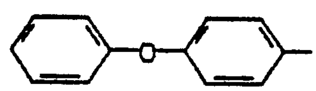 |

Tabelle 1 - Fortsetzung

| $R^1$ | $R^2$ |
|---|---|
| 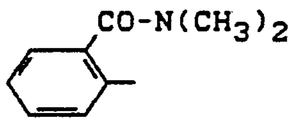 | 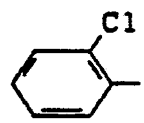 |
| 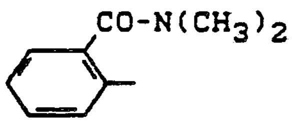 | 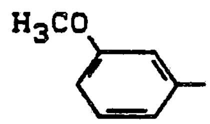 |
| 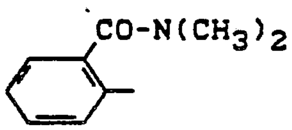 | 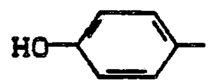 |
| 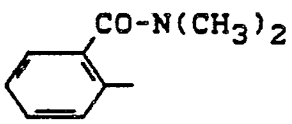 | 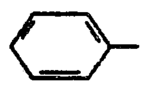 |
| 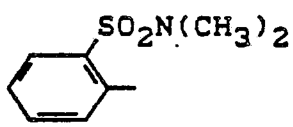 | 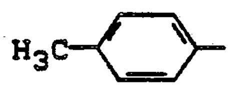 |
| 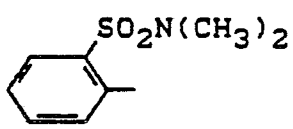 | 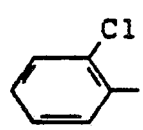 |
| 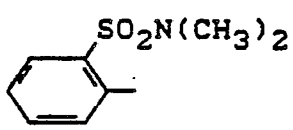 | 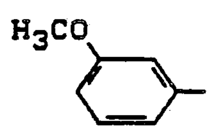 |
| 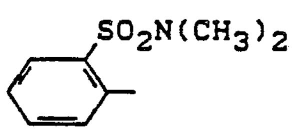 | 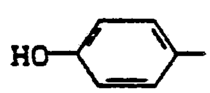 |
| 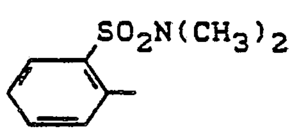 | 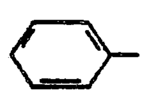 |

Tabelle 1

| $R^1$ | $R^2$ |
|---|---|
| 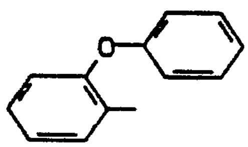 | 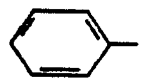 |
| 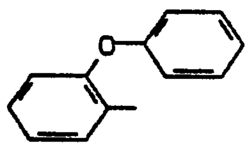 | 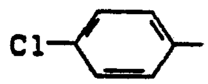 |
| 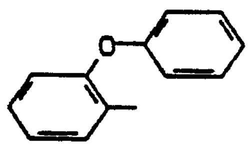 | 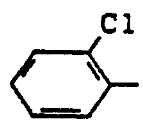 |
| 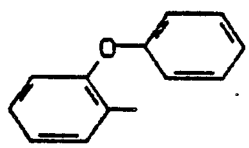 | 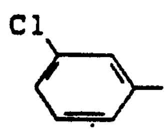 |
| 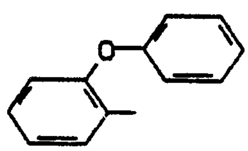 | 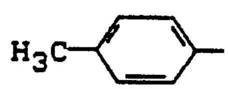 |
| 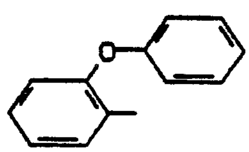 | 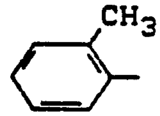 |
| 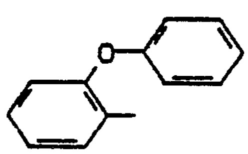 | 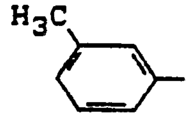 |
| 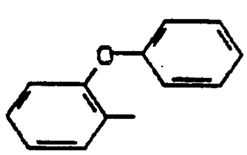 | 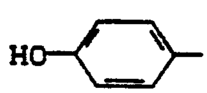 |

Tabelle 1 - Fortsetzung

| $R^1$ | $R^2$ |
|---|---|
| 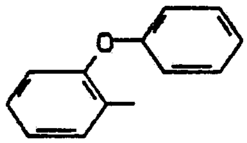 | 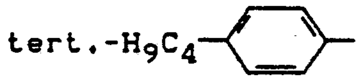 |
| 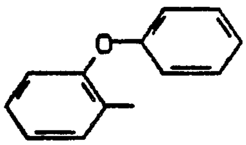 | 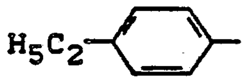 |
| 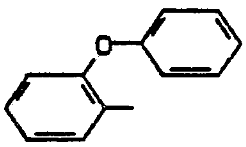 | 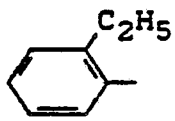 |
| 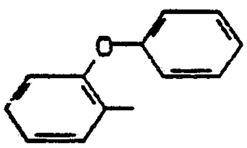 | 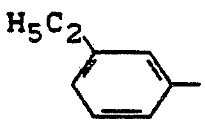 |
| 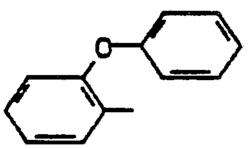 | 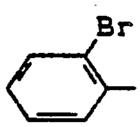 |
| 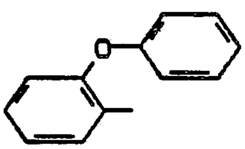 | 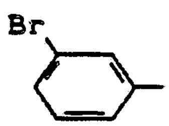 |
| 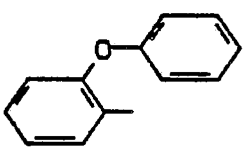 | 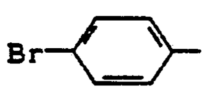 |
| 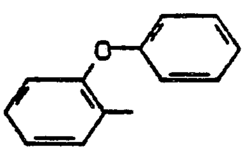 | 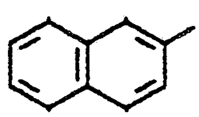 |

Tabelle 1 - Fortsetzung

| $R^1$ | $R^2$ |
|---|---|
| 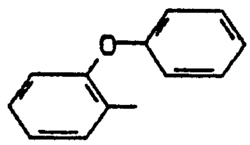 | 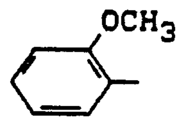 |
| 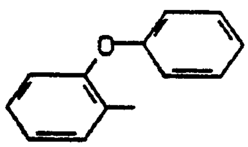 | 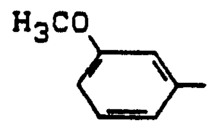 |
| 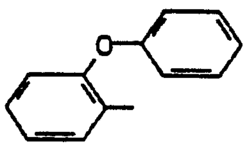 | 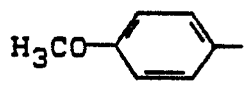 |
| 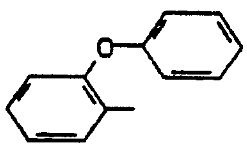 | 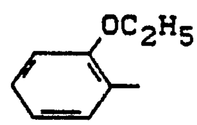 |
| 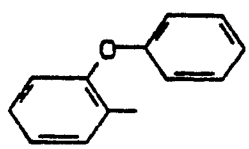 | 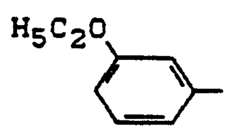 |
| 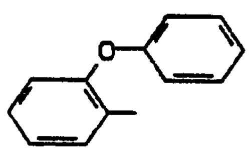 | 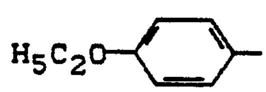 |
| 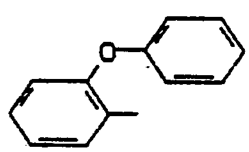 | 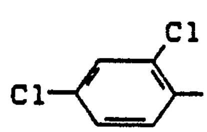 |
| 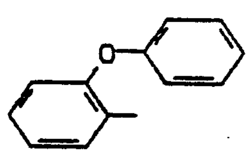 | 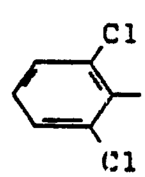 |

<u>Tabelle 1</u> - Fortsetzung

| $R^1$ | $R^2$ |
|---|---|
| 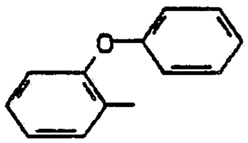 | 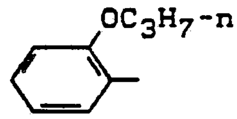 |
| 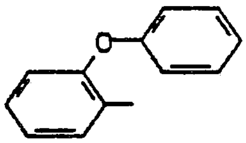 | 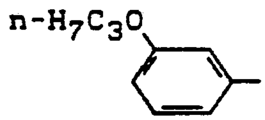 |
| 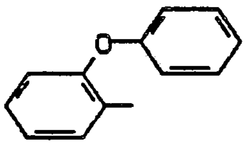 | 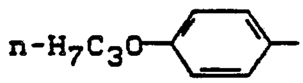 |
| 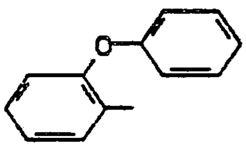 | 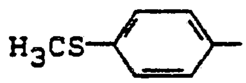 |
| 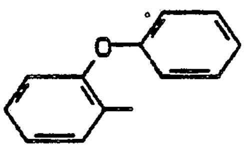 | 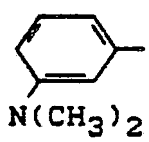 |
| 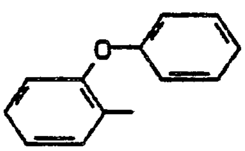 | 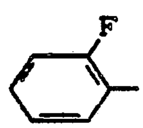 |
| 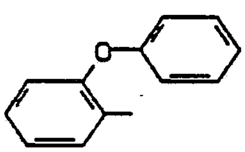 | 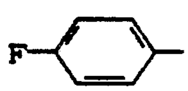 |
| 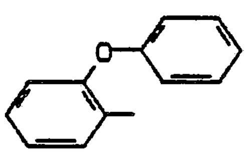 | 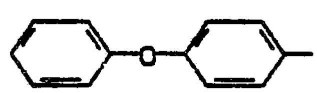 |

Die beim erfindungsgemäßen Verfahren außerdem als Ausgangsstoffe zu verwendenden 2-Aminotriazine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) vorzugsweise für diese Substituenten genannt wurden.

Als Beispiele für die 2-Amino-triazine der Formel (III) seien genannt:

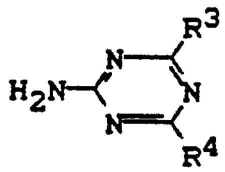

(III)

Tabelle 2

| $R^3$ | $R^4$ |
|---|---|
| $CH_3$ | $CH_3$ |
| $OCH_3$ | $OCH_3$ |
| $CH_3$ | $OCH_3$ |
| $C_2H_5$ | $C_2H_5$ |
| $OC_2H_5$ | $OC_2H_5$ |
| $OC_2H_5$ | $CH_3$ |
| $OC_2H_5$ | $OCH_3$ |
| $CH_3$ | $SCH_3$ |
| $CH_3$ | $SC_2H_5$ |
| $OCH_3$ | $SCH_3$ |
| $OC_2H_5$ | $SCH_3$ |

Tabelle 2 - Fortsetzung

| $R^3$ | $R^4$ |
|---|---|
| $OCH_3$ | $SC_2H_5$ |
| $OC_2H_5$ | $SC_2H_5$ |
| $CH_3$ | $NHCH_3$ |
| $CH_3$ | $N(CH_3)_2$ |
| $CH_3$ | $NHC_2H_5$ |
| $CH_3$ | $N(C_2H_5)_2$ |
| $OCH_3$ | $NHCH_3$ |
| $OCH_3$ | $NHC_2H_5$ |
| $OCH_3$ | $N(CH_3)_2$ |
| $OCH_3$ | $N(C_2H_5)_2$ |
| $OC_2H_5$ | $N(C_2H_5)_2$ |
| $OC_2H_5$ | $N(CH_3)_2$ |
| $SCH_3$ | $NHCH_3$ |
| $SCH_3$ | $N(CH_3)_2$ |
| $SCH_3$ | $NHC_2H_5$ |
| $SCH_3$ | $N(C_2H_5)_2$ |
| $SC_2H_5$ | $NHCH_3$ |
| $SC_2H_5$ | $N(CH_3)_2$ |
| $OC_2H_5$ | $NHCH_3$ |
| $OC_2H_5$ | $NHC_2H_5$ |
| $SC_2H_5$ | $N(C_2H_5)_2$ |
| Cl | $CH_3$ |

Die 2-Aminotriazine der Formel (III) sind bekannt und/oder können nach an sich bekannten Verfahren hergestellt werden.

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methyl-ethyl-, Methyl-isopropyl-und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren werden für das erfindungsgemäße Verfahren zur Herstellung der neuen Verbindungen der Formel (I) alle üblicherweise für derartige Umsetzungen verwendbaren Säurebindemittel eingesetzt. Vorzugsweise in Frage kommen Alkalimetallhydroxide wie z. B. Natrium-und Kaliumhydroxid, Alkalimetallhydride wie z. B. Natriumhydrid, Erdalkalihydroxide wie z. B. Calciumhydroxid, Alkalicarbonate und -alkoholate wie Natrium-und Kaliumcarbonat, Natrium-und Kaliummethylat bzw. -ethylat oder Kalium-tert.-butylat.

Die Reaktionen werden im allgemeinen bei Temperaturen zwischen -20 °C und 150 °C, vorzugsweise bei Temperaturen zwischen 0 °C und 80 °C durchgeführt. Die Reaktionen werden im allgemeinen bei Normaldruck durchgeführt.

Zur Durchführung des erfindungsgemäßen Verfahrens werden die Ausgangsstoffe der Formeln (II) und (III) und gegebenenfalls der geeignete Säureakzeptor in äquimolaren Mengen eingesetzt.

Bevorzugt werden eine Verbindung der Formel (III), der Säureakzeptor und das Verdünnungsmittel wie z. B. Tetrahydrofuran vorgelegt und einige Stunden gerührt. Anschliessend wird die Verbindung der Formel (II) zugegeben. Nach der Umsetzung wird die Reaktionslösung eingeengt, mit Wasser versetzt, über Kieselgel abgesaugt und mit einer Mineralsäure wie z. B. Salzsäure schwach angesäuert. Die Lösung wird in einem organischen Verdünnungsmittel wie z. B. Methylenchlorid aufgenommen. Die organische Phase wird mit gesättigter Natriumcarbonatlösung und anschließend mit Wasser gewaschen, getrocknet und eingeengt. Die Reinigung der Rohprodukte geschieht nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita..

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie-und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht-und Hopfenanlagen und zur selektiven Unkrautbekämpfung in einjährigen Kulturen eingesetzt werden.

Die neuen Wirkstoffe eignen sich zur selektiven Bekämpfung mono-und dikotyler Unkräuter insbesondere in monokotylen Kulturen, wie z. B. Weizen, im Vor-und Nachauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykole sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage; z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z. B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 2-Chlor-N-{[(4-methoxy-6-methyl-1,3,5-triazin-2-yl)-amino]-carbonyl}-benzolsulfonamid, 2-Hydroxycarbonyl-N-{[(4-methoxy-6-methyl-1,3,5-triazin-2-yl)-amino]-carbonyl}-benzolsulfonamid, 2-Ethylamino-6-(1,1-dimethylethyl-amino)-4-methylthio-1,3,5-triazin, 4-Amino-6-(1,1-dimethylethyl)-3-methylthio-1,2,4-triazin-5(4H)-on, 4-Amino-6-(1,1-dimethylethyl)-3-ethylthio-1,2,4-triazin-5(4H)-on, 1-Amino-6-ethylthio-3-(2,2-dimethylpropyl)-1,3,5-triazin-2,4-(1H,3H)-dion, 3-(2,4-Dichlor-phenoxy)-6-nitro-benzoesäuremethylester, 2-[4-(2,4-Dichlorphenoxy)-phenxoy]propionsäuremethylester, das R-Enantiomere des 2-{4-[(3,5-Dichlor-2-pyridinyl)-oxy]-phenoxy}-propionsäure(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methyl-phenoxy)-propionsäure, 3,5-Diiod-4-hydroxy-benzonitril, 3,5-Dibrom-4-hydroxy-benzonitril, N-(1-Ethylpropyl)-3,4-dimethyl-2,6-dinitroanilin, N,N-Diisopropyl-2,3,3-trichlorallylthiocarbamat, 2-(2-Benzthiazolyloxy)-N-methyl-N-phenylacetamid, 2-[4,5-Dihydro-4-methyl-4-(1-methylethyl)-5-oxo-1H-imidazol-2-yl]-4(5)-methyl-benzoesäuremethylester, [(4-Amino-3,5-dichlor-6-fluor-2-pyridyl)-oxy]-essigsäure und 3-Isopropyl-2,1,3-benzothiadiazinon-(4)-2,2-dioxid. Einige Mischungen besitzen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

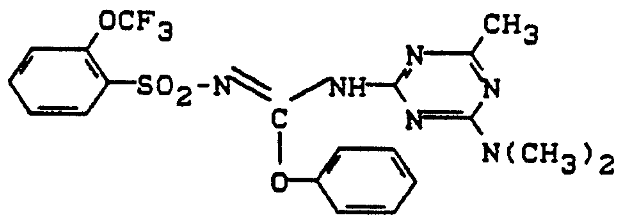

4,1 g (0,027 Mol) 2-Amino-4-dimethylamino-6-methyl-1,3,5-triazin werden in 100 ml Tetrahydrofuran gelöst, mit 3,2 g (0,029 Mol) Kalium-tert.-butylat versetzt und 2 Stunden bei 20 °C gerührt. Anschließend werden 11,8 g (0,027 Mol) N-(2-Trifluormethoxy-benzolsulfonyl)-iminokohlensäure-O,O-diphenylester bei 20 °C zugegeben. Das Reaktionsgemisch wird ca. 15 Stunden bei 20 °C weitergerührt, dann auf 500 ml Eiswasser gegossen, abfiltriert, mit 2-N-Salzsäure angesäuert und mit Methylenchlorid extrahiert. Die organische Phase wird gewaschen, getrocknet und im Vakuum eingeengt. Der Rückstand wird aus Isopropanol umkristallisiert.

Man erhält 9,9 g (73,9 % der Theorie) N'-(4-Dimethylamino-6-methyl-1,3,5-triazin-2-yl)-N''-(2-trifluormethoxybenzolsulfonyl)-O-phenyl-isoharnstoff vom Schmelzpunkt 129 °C.

### Beispiel 2

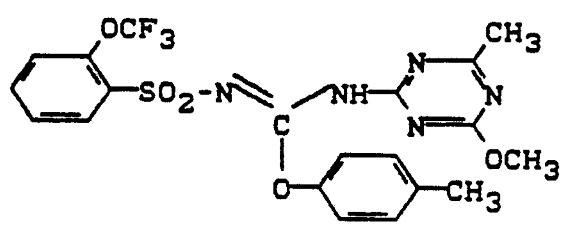

Eine Mischung von 5 g (0.035 Mol) 2-Amino-4-methoxy-6-methyl-1,3,5-triazin, 2,4 g (0,07 Mol) Natriumhydrid in Paraffin und 100 ml Tetrahydrofuran wird 15 Stunden bei 25 °C gerührt. Anschließend wird die Mischung portionsweise mit 15,8 g (0,035 Mol) N-(2-Trifluormethoxybenzolsulfonyl)-iminokohlensäure-O,O-di-(4-methyl-phenyl)-ester in der Weise versetzt, daß eine Temperatur von 30 °C nicht überschritten wird, und 3 Stunden bei 25 °C gerührt. Danach wird das Reaktionsgemisch unter Eiskühlung mit 300 ml Wasser versetzt. Die wäßrige Phase wird mit verdünnter Salzsäure auf einen pH ca. 5 eingestellt, zweimal mit Wasser gewaschen, über Natriumsulfat getrocknet und eingeengt. Nach dem Andestillieren (Badtemperatur: 140 °C, 200 Pa) wird der Rückstand mit Ethanol verrieben, abfiltriert und getrocknet.

Man erhält 6,5 g (36 % der Theorie) N'-(4-Methoxy-6-methyl-1,3,5-triazin-2-yl)-N''-(2-trifluormethoxybenzolsulfonyl)-O-(4-methyl-phenyl)-isoharnstoff in Form einer wachsartigen Substanz.

Analog Beispiel 1 und 2 können die nachfolgenden Verbindungen der Formel (I) hergestellt werden:

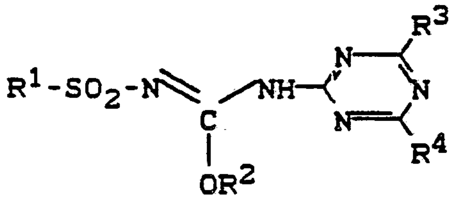 (I)

<u>Tabelle 3</u>

| Beisp.-Nr. | $R^1$ | $R^2$ | $R^3$ | $R^4$ | Fp /[° C] |
|---|---|---|---|---|---|
| 3 | 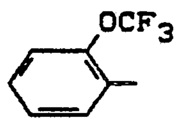 | 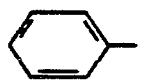 | $CH_3$ | $CH_3$ | 149 |
| 4 | 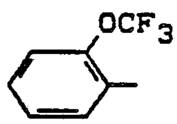 | 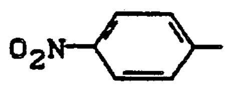 | $CH_3$ | $CH_3$ | |
| 5 | 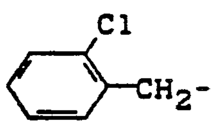 | 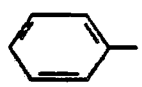 | $CH_3$ | $CH_3$ | 153 |
| 6 | 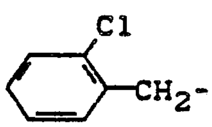 | 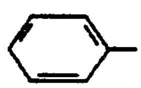 | $CH_3$ | $OCH_3$ | 144 |
| 7 | 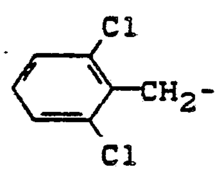 | 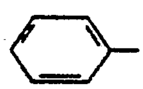 | $CH_3$ | $CH_3$ | 182 |

Tabelle 3 - Fortsetzung

| Beisp.-Nr. | $R^1$ | $R^2$ | $R^3$ | $R^4$ | Fp /[° C] |
|---|---|---|---|---|---|
| 8 | 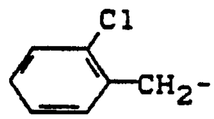 | 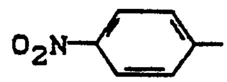 | $CH_3$ | $CH_3$ | 176 |
| 9 | 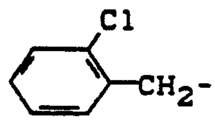 | 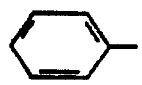 | $CH_3$ | $OCH_3$ | 143 |
| 10 | 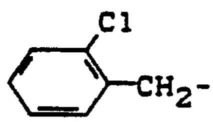 | 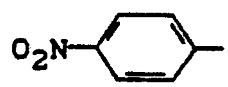 | $CH_3$ | $OCH_3$ | 162 |
| 11 | 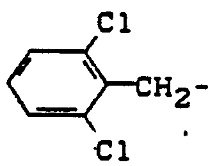 | 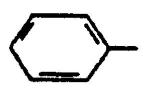 | $CH_3$ | $OCH_3$ | 168 |
| 12 | 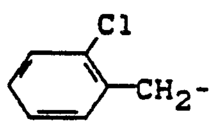 | 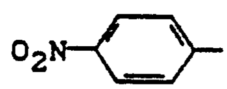 | $OCH_3$ | $OCH_3$ | 171 |
| 13 | 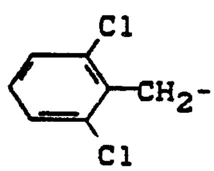 | 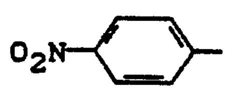 | $CH_3$ | $CH_3$ | 198 |
| 14 | 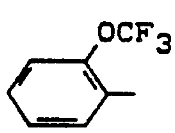 | 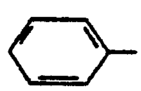 | $CH_3$ | $SCH_3$ | 83 |
| 15 | 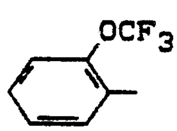 | 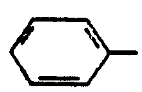 | Cl | $CH_3$ | 132 |

<u>Tabelle 3</u> - Fortsetzung

| Beisp.-<br>Nr. | $R^1$ | $R^2$ | $R^3$ | $R^4$ | Fp<br>/[°C] |
|---|---|---|---|---|---|
| 16 | 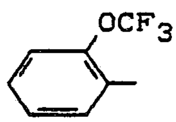 | 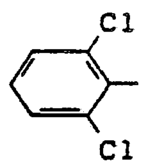 | $OCH_3$ | $OCH_3$ | 131 |
| 17 | 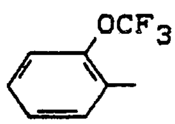 | 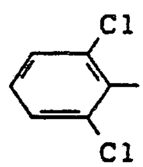 | $CH_3$ | $SCH_3$ | 157 |
| 18 | 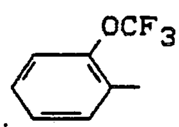 | 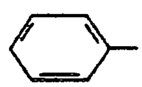 | $CH_3$ | $OCH_3$ | 76 |
| 19 | 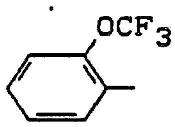 | 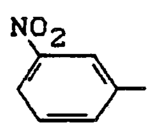 | $OCH_3$ | $OCH_3$ | 152 |
| 20 | 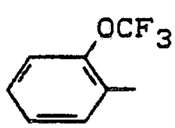 | 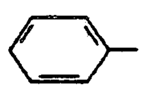 | $OCH_3$ | $OCH_3$ | 111 |
| 21 | 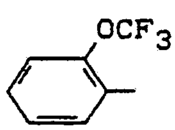 | 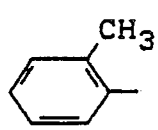 | $CH_3$ | $OCH_3$ | 127 |
| 22 | 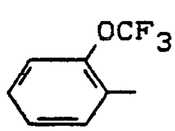 | 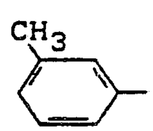 | $CH_3$ | $OCH_3$ | 104 |

<u>Tabelle 3</u> - Fortsetzung

| Beisp.-Nr. | $R^1$ | $R^2$ | $R^3$ | $R^4$ | Fp /[°C] |
|---|---|---|---|---|---|
| 23 | 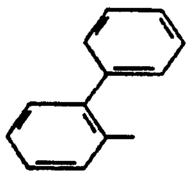 | 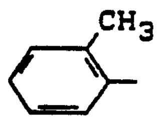 | $CH_3$ | $OCH_3$ | |
| 24 | 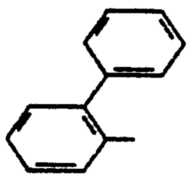 | 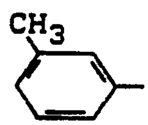 | $CH_3$ | $OCH_3$ | |
| 25 | 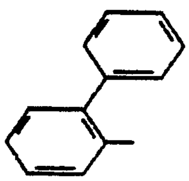 | 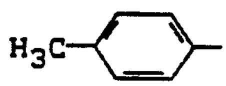 | $CH_3$ | $OCH_3$ | 139 |
| 26 | 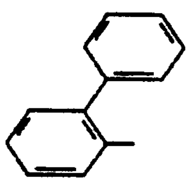 | 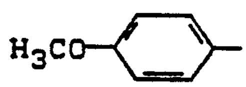 | $CH_3$ | $OCH_3$ | |
| 27 | 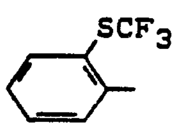 | 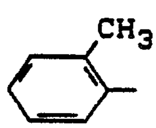 | $CH_3$ | $OCH_3$ | |
| 28 | 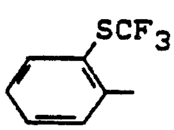 | 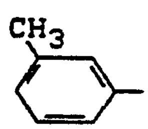 | $CH_3$ | $OCH_3$ | |
| 29 | 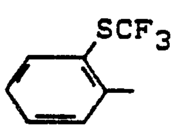 | 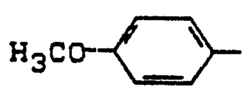 | $CH_3$ | $OCH_3$ | |

Tabelle 3 - Fortsetzung

| Beisp.-Nr. | $R^1$ | $R^2$ | $R^3$ | $R^4$ | Fp /[°C] |
|---|---|---|---|---|---|
| 30 | 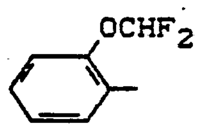 | 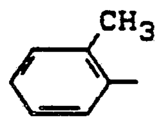 | $CH_3$ | $OCH_3$ | |
| 31 | 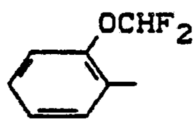 | 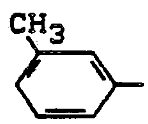 | $CH_3$ | $OCH_3$ | |
| 32 | 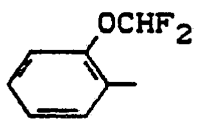 | 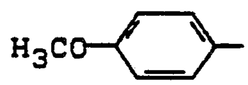 | $CH_3$ | $OCH_3$ | |
| 33 | 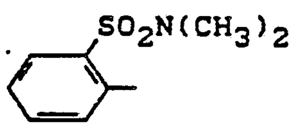 | 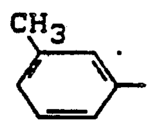 | $CH_3$ | $OCH_3$ | |
| 34 | 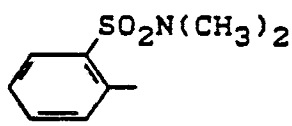 | 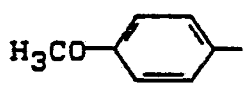 | $CH_3$ | $OCH_3$ | |
| 35 | 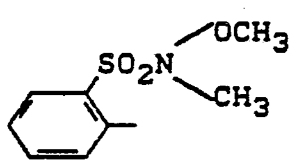 | 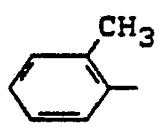 | $CH_3$ | $OCH_3$ | |
| 36 | 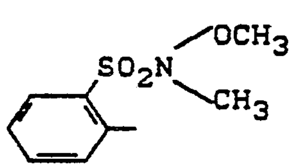 | 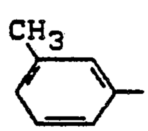 | $CH_3$ | $OCH_3$ | |

Tabelle 3 - Fortsetzung

| Beisp.-Nr. | $R^1$ | $R^2$ | $R^3$ | $R^4$ | Fp /[° C] |
|---|---|---|---|---|---|
| 37 | 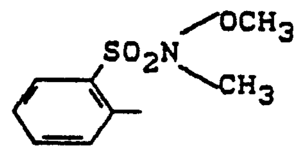 | 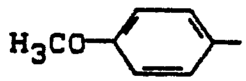 | $CH_3$ | $OCH_3$ | |
| 38 | 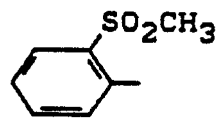 | 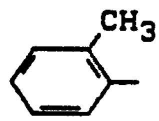 | $CH_3$ | $OCH_3$ | |
| 39 | 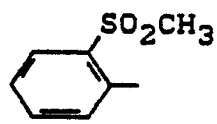 | 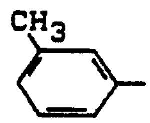 | $CH_3$ | $OCH_3$ | |
| 40 | 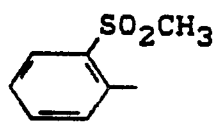 | 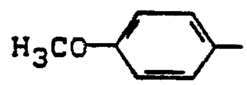 | $CH_3$ | $OCH_3$ | |
| 41 | 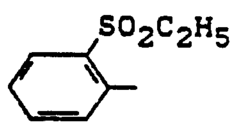 | 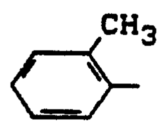 | $CH_3$ | $OCH_3$ | |
| 42 | 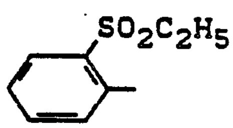 | 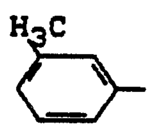 | $CH_3$ | $OCH_3$ | |
| 43 | 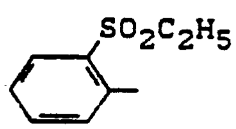 | 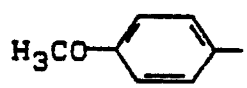 | $CH_3$ | $OCH_3$ | |
| 44 | 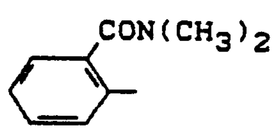 | 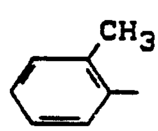 | $CH_3$ | $OCH_3$ | |

Tabelle 3 - Fortsetzung

| Beisp.-Nr. | $R^1$ | $R^2$ | $R^3$ | $R^4$ | Fp /[°C] |
|---|---|---|---|---|---|
| 45 | 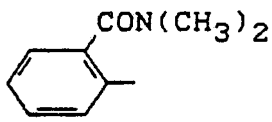 | 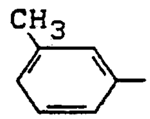 | $CH_3$ | $OCH_3$ | |
| 46 | 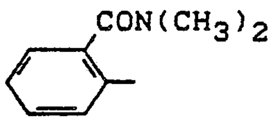 | 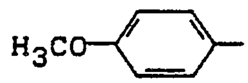 | $CH_3$ | $OCH_3$ | |
| 47 | 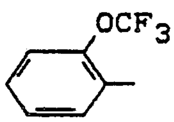 | 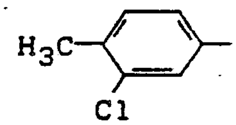 | $CH_3$ | $SCH_3$ | 117 |
| 48 | 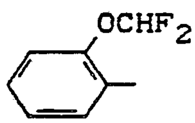 | 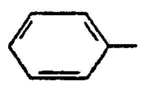 | $CH_3$ | $SCH_3$ | 101 |
| 49 | 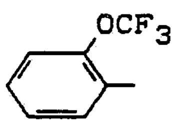 | 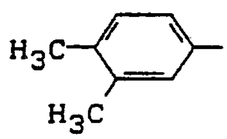 | $OCH_3$ | $OCH_3$ | 100 |
| 50 | 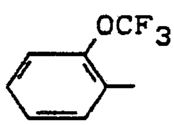 | 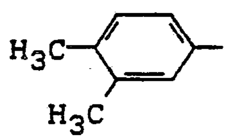 | $CH_3$ | $SCH_3$ | 105 |
| 51 | 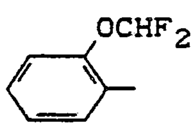 | 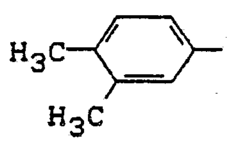 | $CH_3$ | $SCH_3$ | 84 |
| 52 | 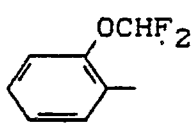 | 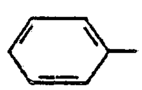 | $CH_3$ | $OCH_3$ | 130 |

Tabelle 3 - Fortsetzung

| Beisp.-Nr. | $R^1$ | $R^2$ | $R^3$ | $R^4$ | Fp /[°C] |
|---|---|---|---|---|---|
| 53 | 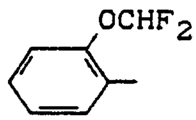 | 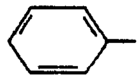 | $OCH_3$ | $OCH_3$ | 106 |
| 54 | 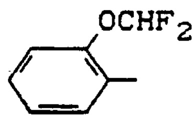 | 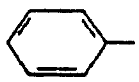 | $CH_3$ | $N(CH_3)_2$ | 113 |
| 55 | 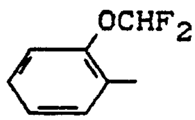 | 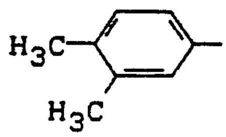 | $OCH_3$ | $OCH_3$ | 126 |
| 56 | 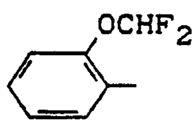 | 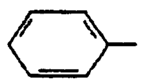 | $CH_3$ | $CH_3$ | 104 |
| 57 | 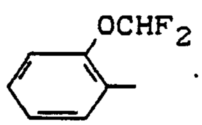 | 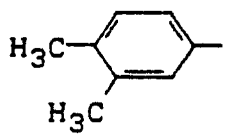 | $CH_3$ | $N(CH_3)_2$ | 141 |
| 58 | 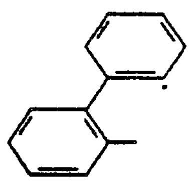 | 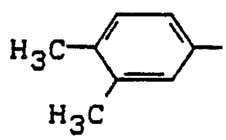 | $OCH_3$ | $OCH_3$ | 134 |
| 59 | 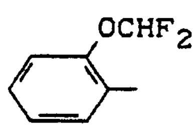 | 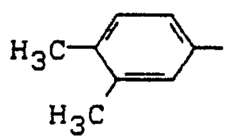 | $CH_3$ | $OCH_3$ | 75 |
| 60 | 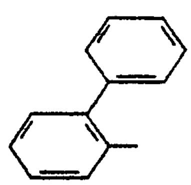 | 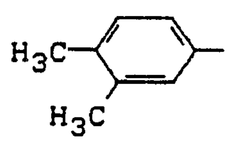 | $CH_3$ | $OCH_3$ | 124 |

Tabelle 3 - Fortsetzung

| Beisp.-Nr. | $R^1$ | $R^2$ | $R^3$ | $R^4$ | Fp /[° C] |
|---|---|---|---|---|---|
| 61 | 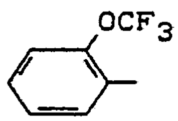 | 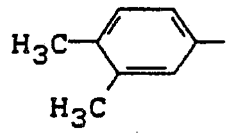 | $CH_3$ | $N(CH_3)_2$ | 147 |
| 62 | 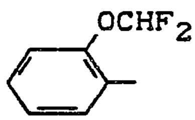 | 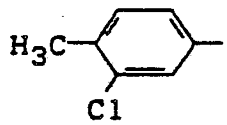 | $CH_3$ | $OCH_3$ | 130 |
| 63 | 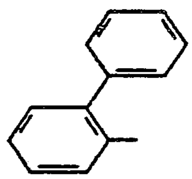 | 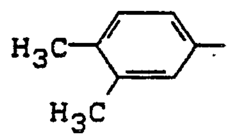 | $CH_3$ | $SCH_3$ | 127 |
| 64 | 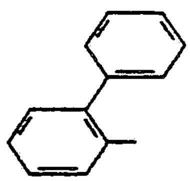 | 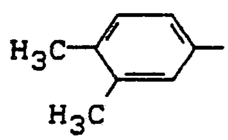 | $CH_3$ | $N(CH_3)_2$ | 171 |
| 65 | 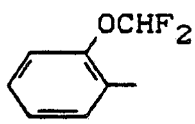 | 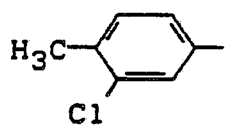 | $CH_3$ | $SCH_3$ | 98 |
| 66 | 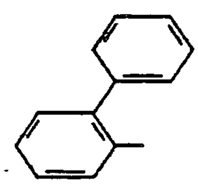 | 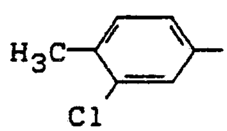 | $OCH_3$ | $OCH_3$ | 131 |
| 67 | 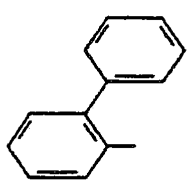 | 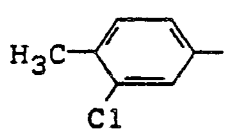 | $CH_3$ | $OCH_3$ | 117 |

Tabelle 3 - Fortsetzung

| Beisp.-Nr. | $R^1$ | $R^2$ | $R^3$ | $R^4$ | Fp /[°C] |
|---|---|---|---|---|---|
| 68 | 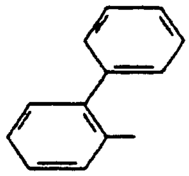 | 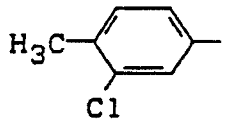 | $CH_3$ | $SCH_3$ | 118 |
| 69 | 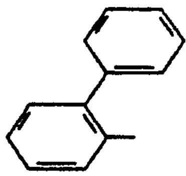 | 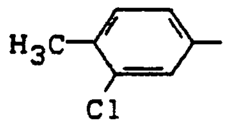 | $CH_3$ | $N(CH_3)_2$ | 172 |

Ausgangsverbindungen der Formel (II)

Beispiel (II-1)

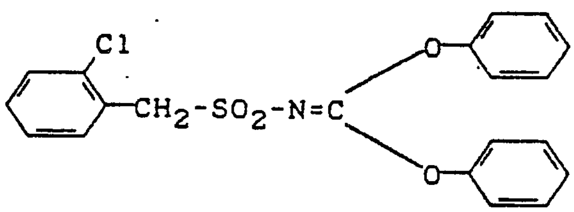

23,1 g (0,08 Mol) 2-Chlor-benzylsulfonylisocyaniddichlorid werden in 200 ml Aceton gelöst und bei 20 °C mit 18,8 g (0,16 Mol) Natriumphenolat versetzt. Die exotherme Reaktion wird gekühlt. Das Reaktionsgemisch wird über Nacht bei Raumtemperatur gerührt, anschließend abgesaugt. Das Filtrat wird im Vakuum eingeengt, der entstandene schmierige Rückstand kristallisiert langsam durch. Der Kristallbrei wird mit Diisopropylether verrieben, abgesaugt, gewaschen und getrocknet.

Man erhält 26,6 g (83 % der Theorie) 2-Chlor-benzylsulfonyliminokohlensäure-O,O-diphenylester vom Schmelzpunkt 130 °C.

Beispiel (II-2)

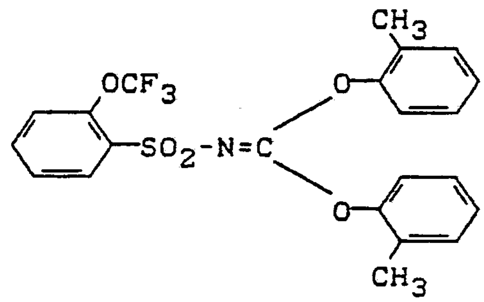

Eine Mischung aus 21,6 g (0,2 Mol) 2-Methylphenol und 14 g (0,2 Mol) Kaliumcarbonat in 200 ml Acetonitril wird 30 Minuten auf 80 °C erhitzt und abgekühlt. In diese Mischung werden 32,2 g (0,1 Mol) 2-Trifluormethoxybenzolsulfonylisocyaniddichlorid in der Weise getropft, daß eine Reaktionstemperatur von 50 °C nicht überschritten wird; anschließend wird 18 Stunden bei 25 °C nachgerührt. Das Reaktionsgemisch wird mit 500 ml Wasser verdünnt und zweimal mit jeweils 100 ml Methylenchlorid extrahiert. Die Methylenchloridlösung wird mit 100 ml 5 %iger Natronlauge und 100 ml Wasser gewaschen und anschließend eingeengt.

Man erhält 35 g (75 % der Theorie) 2-Trifluormethoxybenzolsulfonyliminokohlensäure-O,O-di-(2-methylphenyl)-ester in Form farbloser Kristalle vom Schmelzpunkt 95 °C.

Analog Beipiel (II-1) und (II-2) können die folgenden Verbindungen der Formel (II) hergestellt werden:

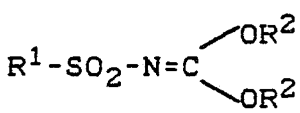 (II)

Tabelle 4

| Beisp.-Nr. | $R^1$ | $R^2$ | Physikal. Konstante |
|---|---|---|---|
| II-3 | 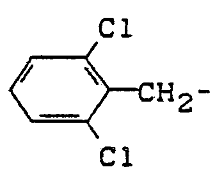 | 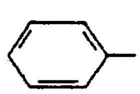 | Fp. 177 °C |

Tabelle 4 - Fortsetzung

| Beisp.-Nr. | $R^1$ | $R^2$ | Physikal. Konstante |
|---|---|---|---|
| II-4 | 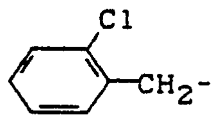 | 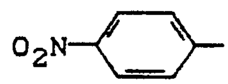 | Fp. 185 °C |
| II-5 | 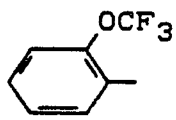 | 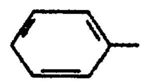 | Fp. 112 °C |
| II-6 | 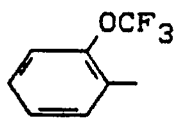 | 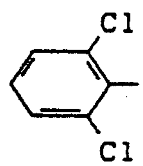 | Fp. 184 °C |
| II-7 | 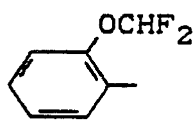 | 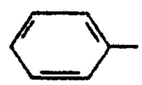 | |
| II-8 | 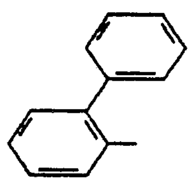 | 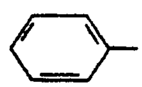 | |
| II-9 | 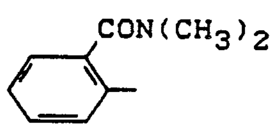 | 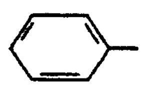 | |
| II-10 | 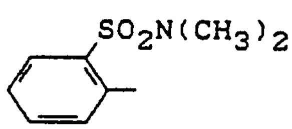 | 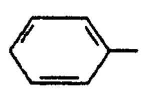 | |
| II-11 | 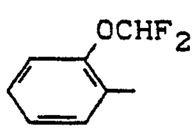 | 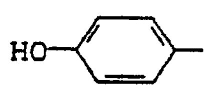 | |

Tabelle 4 - Fortsetzung

| Beisp.-Nr. | $R^1$ | $R^2$ | Physikal. Konstante |
|---|---|---|---|
| II-12 | 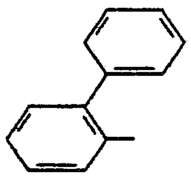 | 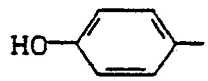 | |
| II-13 | 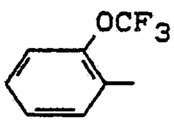 | 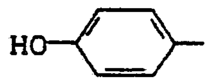 | |
| II-14 | 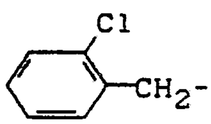 | 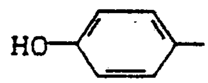 | |
| II-15 | 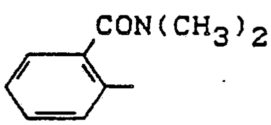 | 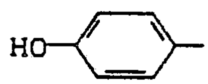 | |
| II-16 | 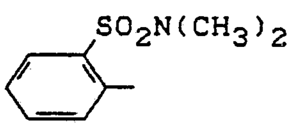 | 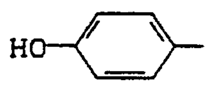 | |
| II-17 | 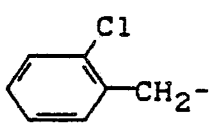 | 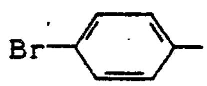 | |
| II-18 | 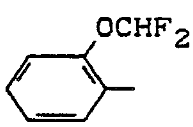 | 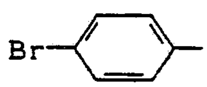 | |
| II-19 | 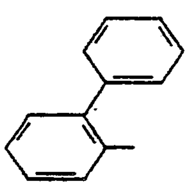 | 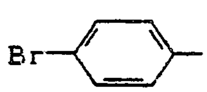 | |

Tabelle 4 - Fortsetzung

| Beisp.-Nr. | $R^1$ | $R^2$ | Physikal. Konstante |
|---|---|---|---|
| II-20 | 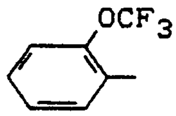 | 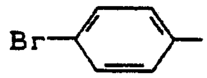 | |
| II-21 | 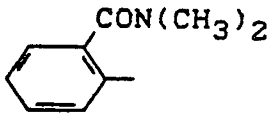 | 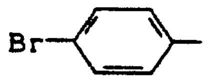 | |
| II-22 | 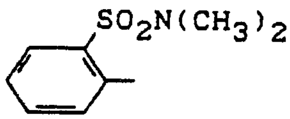 | 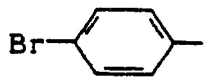 | |
| II-23 | 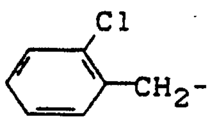 | 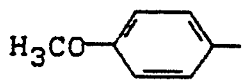 | |
| II-24 | 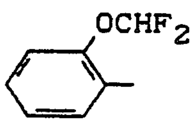 | 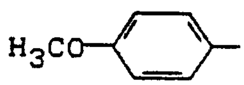 | |
| II-25 | 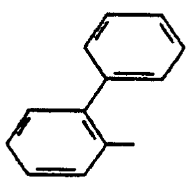 | 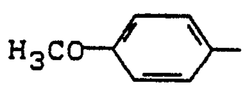 | Wachs |
| II-26 | 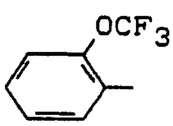 | 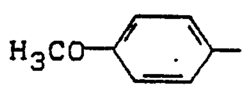 | Fp. 113 °C |
| II-27 | 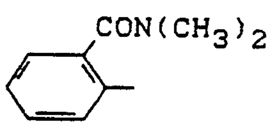 | 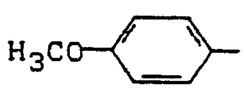 | |

Tabelle 4 - Fortsetzung

| Beisp.-Nr. | $R^1$ | $R^2$ | Physikal. Konstante |
|---|---|---|---|
| II-28 | 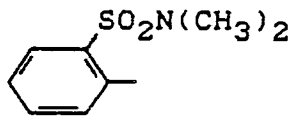 | 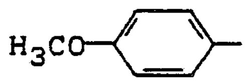 | |
| II-29 | 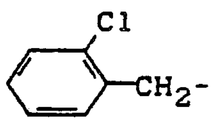 | 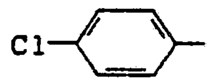 | |
| II-30 | 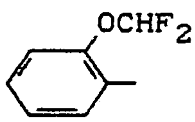 | 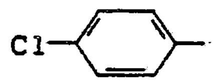 | |
| II-31 | 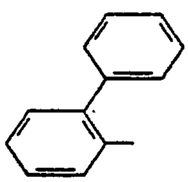 | 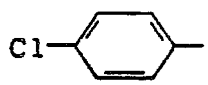 | |
| II-32 | 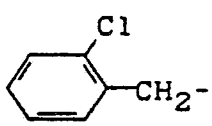 | 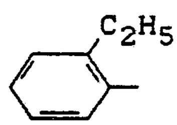 | |
| II-33 | 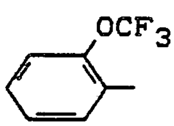 | 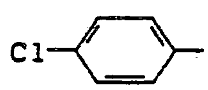 | |
| II-34 | 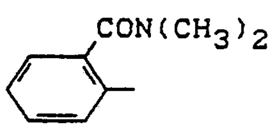 | 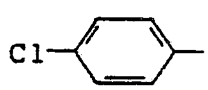 | |
| II-35 | 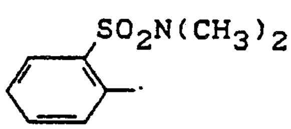 | 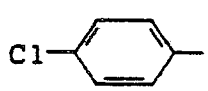 | |

Tabelle 4 - Fortsetzung

| Beisp.-Nr. | $R^1$ | $R^2$ | Physikal. Konstante |
|---|---|---|---|
| II-36 | 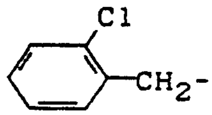 | 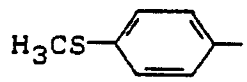 | |
| II-37 | 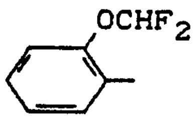 | 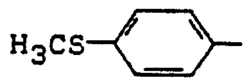 | |
| II-38 | 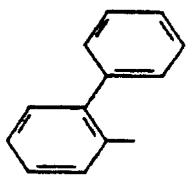 | 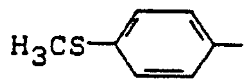 | |
| II-39 | 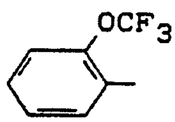 | 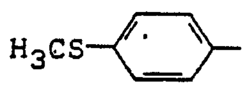 | |
| II-40 | 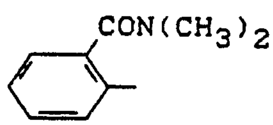 | 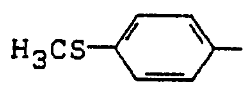 | |
| II-41 | 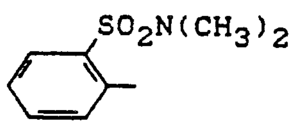 | 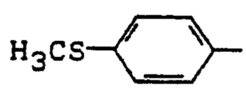 | |
| II-42 | 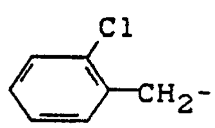 | 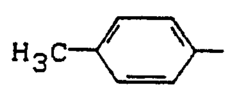 | |
| II-43 | 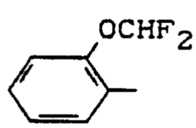 | 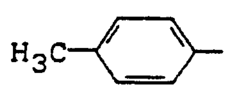 | |

Tabelle 4 - Fortsetzung

| Beisp.-Nr. | $R^1$ | $R^2$ | Physikal. Konstante |
|---|---|---|---|
| II-44 | 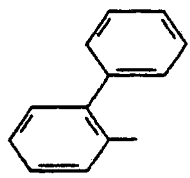 | 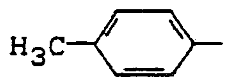 | Fp. 160 °C |
| II-45 | 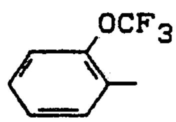 | 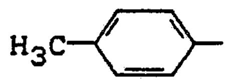 | Fp. 92-94 °C |
| II-46 | 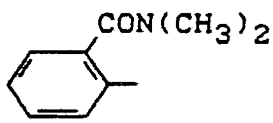 | 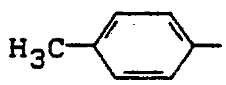 | |
| II-47 | 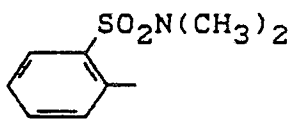 | 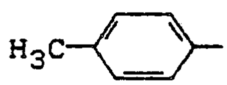 | |
| II-48 | 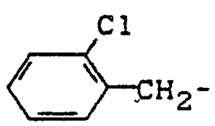 | 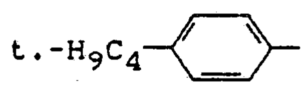 | 106 °C |
| II-49 | 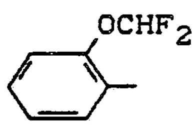 | 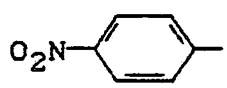 | |
| II-50 | 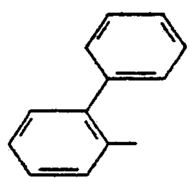 | 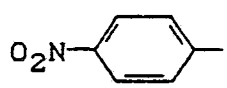 | |
| II-51 | 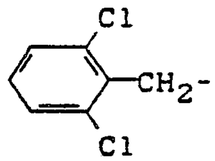 | 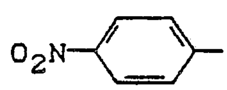 | |

Tabelle 4 - Fortsetzung

| Beisp.-Nr. | $R^1$ | $R^2$ | Physikal. Konstante |
|---|---|---|---|
| II-52 | 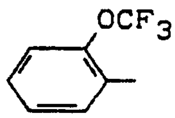 | 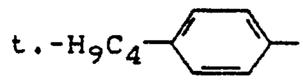 | Fp. 119 °C |
| II-53 | 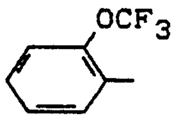 | 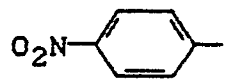 | Fp. 154 °C |
| II-54 | 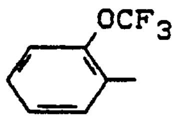 | 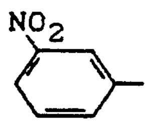 | Fp. 189 °C |
| II-55 | 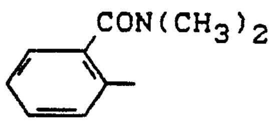 | 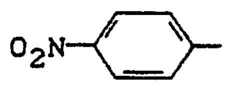 | |
| II-56 | 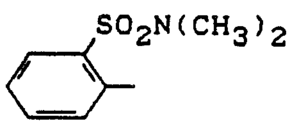 | 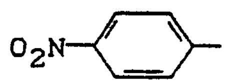 | |
| II-57 | 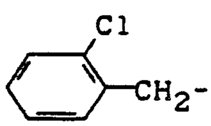 | 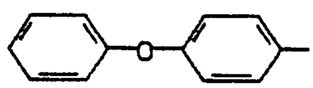 | |
| II-58 | 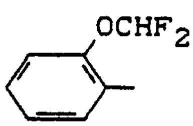 | 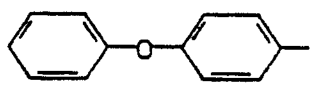 | |
| II-59 | 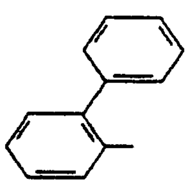 | 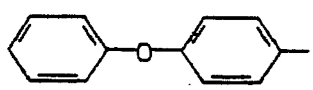 | |

<u>Tabelle 4</u> - Fortsetzung

| Beisp.-Nr. | $R^1$ | $R^2$ | Physikal. Konstante |
|---|---|---|---|
| II-60 | 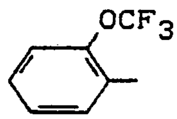 | 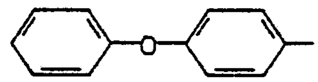 | |
| II-61 | 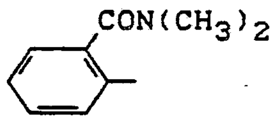 | 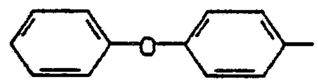 | |
| II-62 | 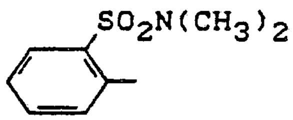 | 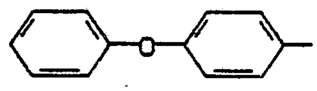 | |
| II-63 | 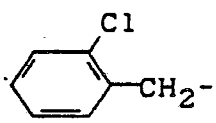 | 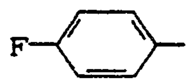 | |
| II-64 | 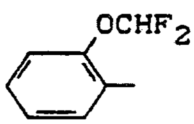 | 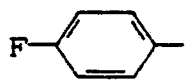 | |
| II-65 | 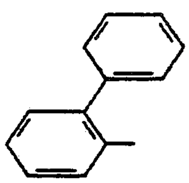 | 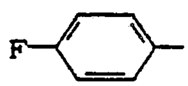 | |
| II-66 | 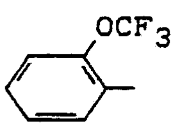 | 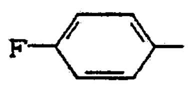 | |
| II-67 | 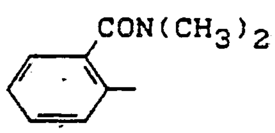 | 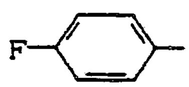 | |

Tabelle 4 - Fortsetzung

| Beisp.-Nr. | $R^1$ | $R^2$ | Physikal. Konstante |
|---|---|---|---|
| II-68 | 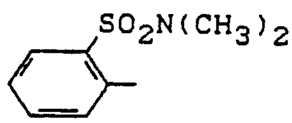 | 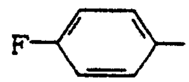 | |
| II-69 | 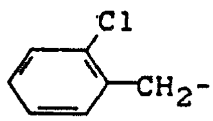 | 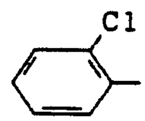 | |
| II-70 | 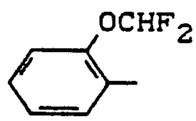 | 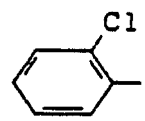 | |
| II-71 | 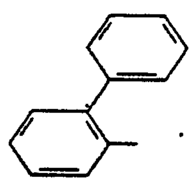 | 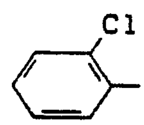 | |
| II-72 | 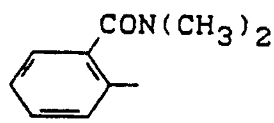 | 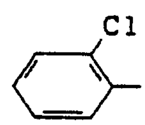 | |
| II-73 | 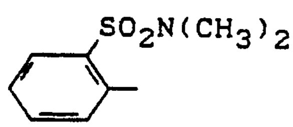 | 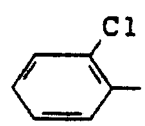 | |
| II-74 | 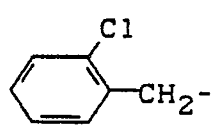 | 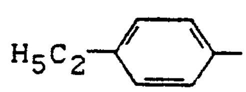 | |
| II-75 | 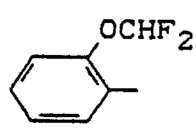 | 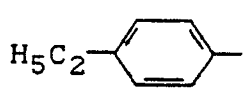 | |

<u>Tabelle 4</u> - Fortsetzung

| Beisp.-Nr. | $R^1$ | $R^2$ | Physikal. Konstante |
|---|---|---|---|
| II-76 | 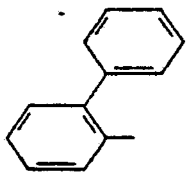 | 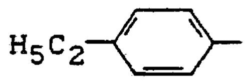 | |
| II-77 | 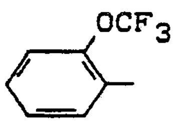 | 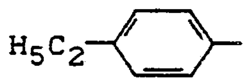 | |
| II-78 | 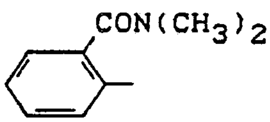 | 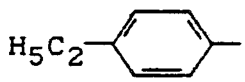 | |
| II-79 | 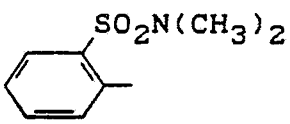 | 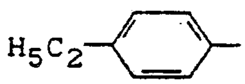 | |
| II-80 | 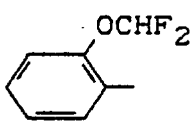 | 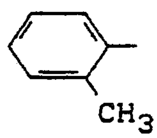 | |
| II-81 | 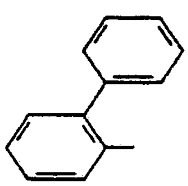 | 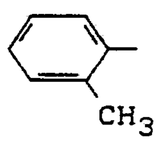 | Wachs |
| II-82 | 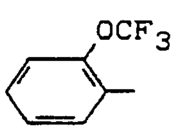 | 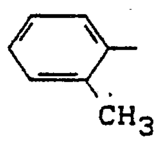 | |

Tabelle 4 - Fortsetzung

| Beisp.-Nr. | $R^1$ | $R^2$ | Physikal. Konstante |
|---|---|---|---|
| II-83 | 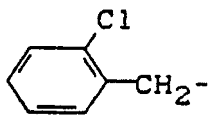 | 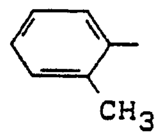 | |
| II-84 | 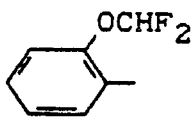 | 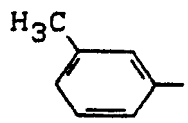 | |
| II-85 | 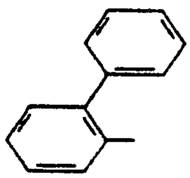 | 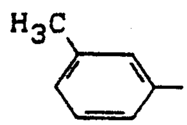 | Wachs |
| II-86 | 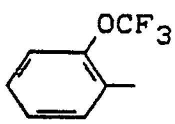 | 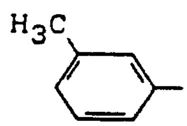 | Fp. 104 °C |
| II-87 | 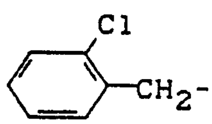 | 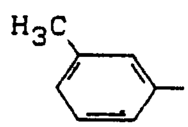 | |
| II-88 | 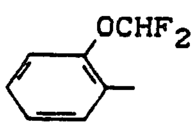 | 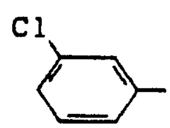 | |
| II-89 | 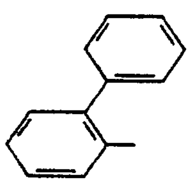 | 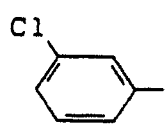 | |
| II-90 | 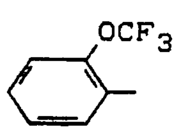 | 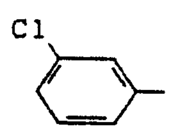 | |

Tabelle 4 - Fortsetzung

| Beisp.-Nr. | $R^1$ | $R^2$ | Physikal. Konstante |
|---|---|---|---|
| II-91 | 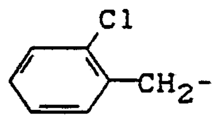 | 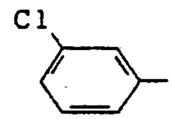 | |
| II-92 | 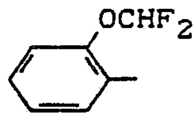 | 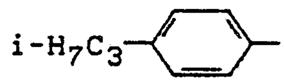 | |
| II-93 | 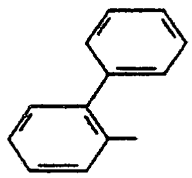 | 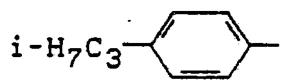 | |
| II-94 | 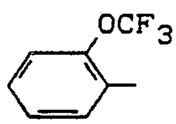 | 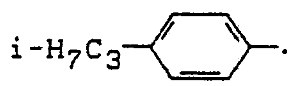 | |
| II-95 | 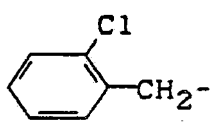 | 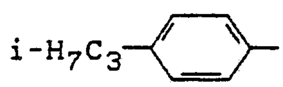 | |
| II-96 | 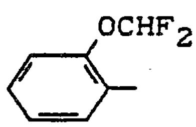 | 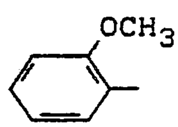 | |
| II-97 | 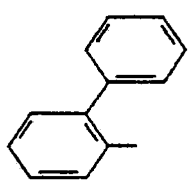 | 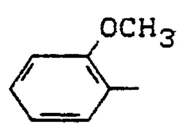 | |
| II-98 | 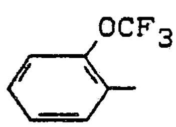 | 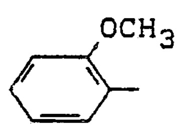 | |

Tabelle 4 - Fortsetzung

| Beisp.-Nr. | $R^1$ | $R^2$ | Physikal. Konstante |
|---|---|---|---|
| II-99 | 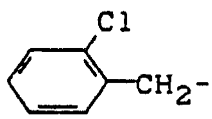 | 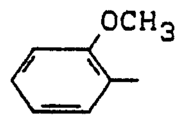 | |
| II-100 | 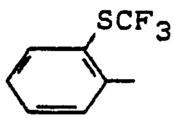 | 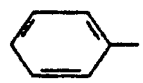 | |
| II-101 | 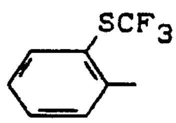 | 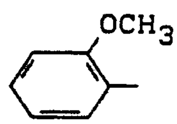 | |
| II-102 | 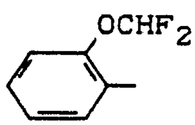 | 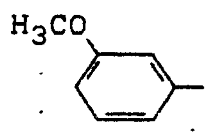 | |
| II-103 | 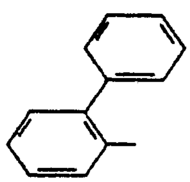 | 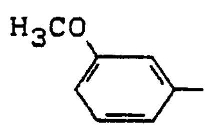 | |
| II-104 | 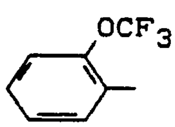 | 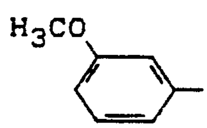 | |
| II-105 | 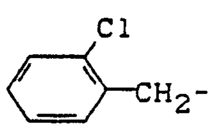 | 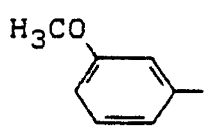 | |
| II-106 | 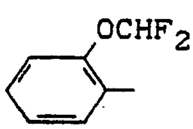 | 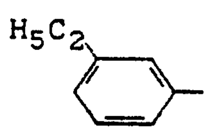 | |

Tabelle 4 - Fortsetzung

| Beisp.-Nr. | $R^1$ | $R^2$ | Physikal. Konstante |
|---|---|---|---|
| II-107 | 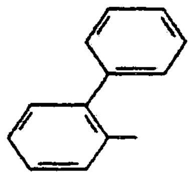 | 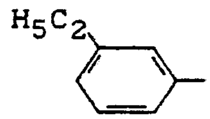 | |
| II-108 | 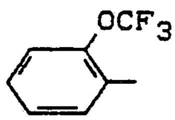 | 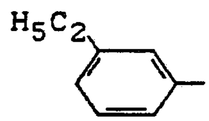 | |
| II-109 | 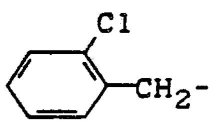 | 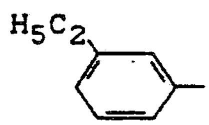 | |
| II-110 | 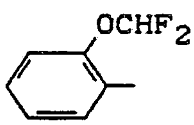 | 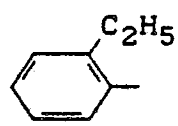 | |
| II-111 | 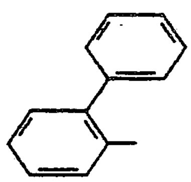 | 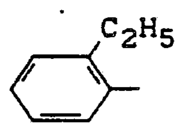 | |
| II-112 | 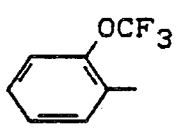 | 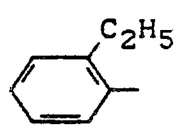 | |

Ausgangsverbindungen der Formel (IV)

Beispiel (IV-1)

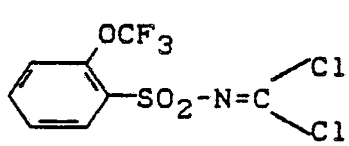

34,5 g (0,1 Mol) 2-Trifluormethoxy-benzolsulfonyl-iminodithiokohlensäure-S,S-dimethylester werden in 300 ml Tetrachlorkohlenstoff gelöst und bei 20 °C wird mit 8 Mol trockenem Chlor versetzt. Das Reaktionsgemisch erwärmt sich dabei auf etwa 40 °C. Es wird 1 Stunde nachgerührt, abfiltriert, das Filtrat im Vakuum eingeengt und der Rückstand im Vakuum fraktioniert destilliert.

Man erhält so 23,2 g (72,4 % der Theorie) 2-Trifluormethoxy-benzolsulfonyl-isocyaniddichlorid vom Siedepunkt Kp. 126 °C/1 mbar.

Die Verbindung des Beispiels (IV-1) läßt sich auch folgendermaßen herstellen:

418 g (3,1 Mol) Sulfurylchlorid werden bei 50 °C portionsweise mit 69 g (0,2 Mol) 2-Trifluormethoxybenzolsulfonyliminodithiokohlensäure-S,S-dimethylester versetzt und anschließend noch 3 Stunden unter Rückfluß erhitzt. Das Reaktionsgemisch wird eingeengt und der Rückstand destilliert.

Man erhält 33 g (50 % der Theorie) 2-Trifluormethoxyben zolsulfonylisocyaniddichlorid mit einem Siedepunkt Kp.: 124 °C/ 101 Pa.

Beispiel (IV-2)

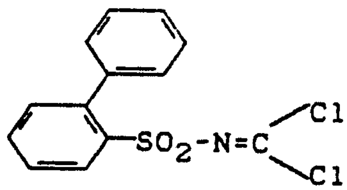

750 g (5,55 Mol) Sulfurylchlorid werden bei 50 °C portionsweise mit 87 g (0,37 Mol) 2-Phenylbenzolsulfonyliminodithiokohlensäure-S,S-dimethylester versetzt. Nach beendeter Zugabe wird noch 3 Stunden unter Rückfluß erhitzt und das Reaktionsgemisch eingedampft.

Nach dem Andestillieren erhält man 67 g (57,9 % der Theorie) 2-Phenylbenzolsulfonylisocyaniddichlorid in Form einer wachsartigen Substanz.

Analog Beispiel (IV-1 und (IV-2) können die folgenden Verbindungen der Formel (IV) erhalten werden:

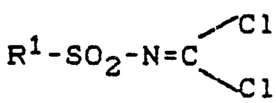

(IV)

Tabelle 5

| Beisp.-Nr. | $R^1$ | Physikal. Konstante |
|---|---|---|
| IV-3 | 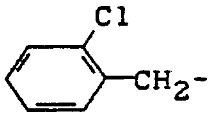 | Fp. 79 °C |
| IV-4 | 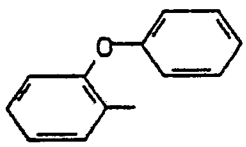 | |
| IV-5 | 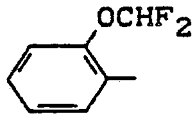 | Kp. 151 °C/101 Pa. |
| IV-6 | 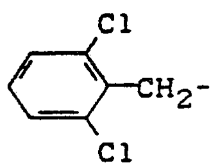 | Fp. 94 °C |
| IV-7 | 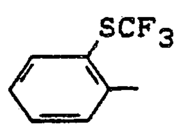 | |

Ausgangsverbindungen der Formel (VI)

Beispiel (VI-1)

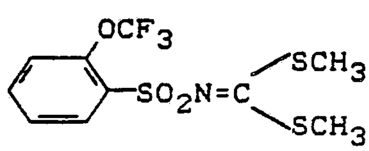

Eine Mischung aus 240 g (1 Mol) 2-Trifluormethoxybenzolsulfonsäureamid, 1500 ml Dimethylformamid und 85 g (1,11 Mol) Schwefelkohlenstoff wird auf 5 °C abgekühlt und mit einer Lösung von 80 g (2 Mol) Natriumhydroxid in 150 ml Wasser in der Weise versetzt, daß eine Reaktionstemperatur von 10 °C nicht überschritten wird. Anschließend wird noch 30 Minuten bei 5 °C - 10 °C gerührt, 284 g (2 Mol) Methyliodid zugetropft und 18 Stunden bei 20 °C -25 °C nachgerührt. Die Reaktionsmischung wird in 5 l Wasser gegossen und das ausgefallene Reaktionsprodukt wird abfiltriert. Der Filterrückstand wird in 1,5 l Methylenchlorid aufgenommen, filtriert, über Natriumsulfat getrocknet und eingeengt. Der Rückstand wird mit Petrolether verrieben.

Man erhält 261 g (85 % der Theorie) 2-Trifluormethoxybenzolsulfonyliminodithiokohlensäure-S,S-dimethylester vom Schmelzpunkt 107 °C.

Analog Beispiel (VI-1) können die in der folgenden Tabelle 6 aufgeführten Verbindungen hergestellt werden:

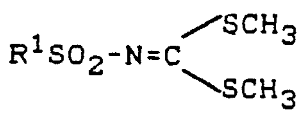 (VI)

Tabelle 6

| Beisp.-Nr. | $R^1$ | Schmelzpunkt / [°C] |
|---|---|---|
| VI-2 | 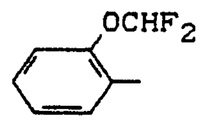 | 103 |
| VI-3 | 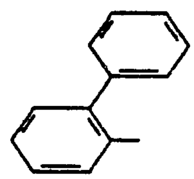 | 117 |
| VI-4 | 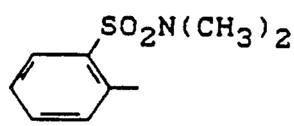 | |
| VI-5 | 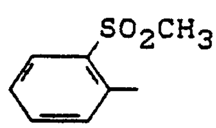 | |
| VI-6 | 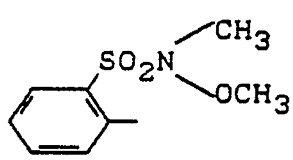 | |

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Fläzcheneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Bei diesem Test zeigt die Verbindung (20) der Hestellungsbeispiele eine bessere herbizide Wirkung gegen Unkräuter wie z. B. Datura, Ipomoea, Portulak, Sinapis und Alopercurus in Kulturen wie z. B. Weizen als die Vergleichsverbindung (A). (A) = N'-(2-Chlor-benzolsulfonyl)-N''-(4,6-dimethyl-1,3,5-triazin-2-yl)-O-phenylisoharnstoff (bekannt aus EP-A 173 957).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigt die Verbindung (20) der Herstellungsbeispiele gegen Unkräuter wie z. B. Cassia, Datura, Matricaria, Sida, Stellaria und Agropyron eine bessere herbizide Wirkung in Kulturen wie z. B. Weizen als die Vergleichsverbindungen (A) und (B). (B) = N'-(2-Chlor-benzolsulfonyl)-N''-(4,6-dimethyl-pyrimidin-2-yl)-O-(4-chlorphenyl)-isoharnstoff (bekannt aus CH-PS 646 957).

**Ansprüche**

1) O-Ary-N'-(triazin-2-yl)-isoharnstoffe der allgemeinen Formel (I)

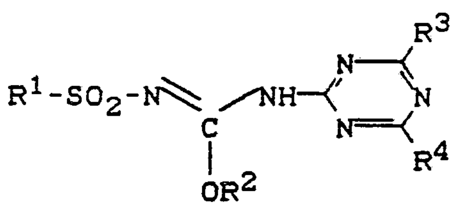

(I)

in welcher
$R^1$ für den Rest

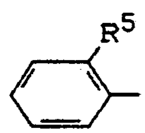

steht, worin
$R^5$ für Trifluormethoxy, Difluormethoxy, Phenyl, Phenoxy, Trifluormethylthio, Difluormethylthio, Di-($C_1$-$C_4$-alkyl)-aminocarbonyl, $C_1$-$C_4$-Alkylsulfonyl, $C_1$-$C_4$-Alkoxyaminosulfonyl, N-($C_1$-$C_4$-Alkoxy)-N-($C_1$-$C_4$-alkyl)-aminosulfonyl, $C_1$-$C_4$-Alkylaminosulfonyl oder Di-($C_1$-$C_4$-alkyl)-aminosulfonyl steht,
worin weiter
$R^1$ für den Rest

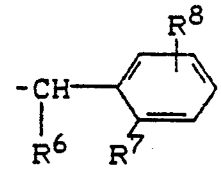

steht, worin

$R^6$ für Wasserstoff oder $C_1$-$C_4$-Alkyl steht,

$R^7$ und $R^8$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welche gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen;

worin weiter

$R^2$ für gegebenenfalls substituiertes Aryl steht,

$R^3$ für Wasserstoff, Halogen, Hydroxy, $C_1$-$C_6$-Alkylamino, Di-($C_1$-$C_6$-alkyl)-amino oder für gegebenenfalls substituiertes $C_1$-$C_6$-Alkyl, $C_1$-$C_6$-Alkoxy und $C_1$-$C_6$-Alkylthio steht und

$R^4$ für Wasserstoff, Halogen, Hydroxy, für gegebenenfalls substituiertes $C_1$-$C_6$-Alkyl, $C_1$-$C_6$-Alkoxy und $C_1$-$C_6$-Alkylthio, für $C_1$-$C_6$-Alkylamino oder Di-($C_1$-$C_6$-alkyl)-amino steht.

2) O-Ary-N'-(triazin-2-yl)-isoharnstoffe der Formel (I) gemäß Anspruch 1, dadurch gekennzeichnet, daß darin

$R^1$ für den Rest

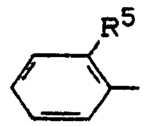

steht, worin

$R^5$ für Trifluormethoxy, Difluormethoxy, Phenyl, Phenoxy, Trifluormethylthio, Difluormethylthio, Di-($C_1$-$C_4$-alkyl)-aminocarbonyl, $C_1$-$C_3$-Alkylsulfonyl, $C_1$-$C_4$-Alkoxyaminosulfonyl, N-($C_1$-$C_2$-Alkoxy)-N-($C_1$-$C_2$-alkyl)-aminosulfonyl, $C_1$-$C_2$-Alkylaminosulfonyl oder Di-($C_1$-$C_2$-alkyl)-aminosulfonyl steht,

worin weiter

$R^1$ für den Rest

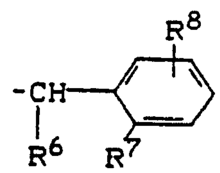

steht, worin

$R^6$ für Wasserstoff oder $C_1$-$C_4$-Alkyl steht,

$R^7$ und $R^8$ gleich oder verschieden sind und für Wasserstoff, Fluor, Chlor, Brom, Cyano, Methyl, Methoxy, Trifluormethyl, Trifluormethoxy, Difluormethoxy, Carboxy, Methoxycarbonyl, Ethoxycarbonyl, Methylsulfonyl, Ethylsulfonyl, Dimethylaminosulfonyl und Diethylaminosulfonyl stehen;

worin weiter

$R^2$ für einen Phenylrest steht, welcher gegebenenfalls substituiert ist durch einen oder mehrere Reste aus der Reihe

Halogen [wie insbesondere Fluor, Chlor, Brom und Iod], Cyano, Nitro, Hydroxy, Carboxy, $C_1$-$C_6$-Alkyl [welches gegebenenfalls durch Fluor, Chlor, Brom, Nitro, Cyano, Hydroxy, Carboxy, $C_1$-$C_4$-Alkoxy-carbonyl, $C_1$-$C_4$-Alkoxy, $C_1$-$C_4$-Alkylthio oder Phenyl substituiert ist], $C_3$-$C_6$-Cycloalkyl, $C_1$-$C_4$-Alkoxy [welches gegebenenfalls durch Fluor, Chlor, Brom, Cyano, Carboxy, $C_1$-$C_4$-Alkoxy, $C_1$-$C_4$-Alkylthio oder $C_1$-$C_4$-Alkoxycarbonyl substituiert ist], $C_1$-$C_4$-Alkylthio [welches gegebenenfalls durch Fluor, Chlor, Brom, Cyano, Carboxy, $C_1$-$C_4$-Alkoxy-carbonyl substituiert ist], Amino, $C_1$-$C_4$-Alkyl-amino bzw. Di-($C_1$-$C_4$-alkyl)-amino [welche gegebenenfalls durch Fluor, Chlor, Brom, Cyano, Carboxy, $C_1$-$C_4$-Alkoxy oder $C_1$-$C_4$-Alkoxy-carbonyl substituiert sind], $C_1$-$C_4$-Alkyl-carbonylamino, $C_1$-$C_4$-Alkoxy-carbonylamino, (Di)-$C_1$-$C_4$-Alkylamino-carbonyl-amino, Formyl, $C_1$-

$C_4$-Alkyl-carbonyl, Benzoyl, $C_1$-$C_4$-Alkoxy-carbonyl, Phenoxy-carbonyl, Benzyloxycarbonyl, Phenyl [welches gegebenenfalls durch Fluor, Chlor, Brom, Cyano, Nitro, Hydroxy oder Methyl substituiert ist], Phenoxy, Phenylthio, Phenylsulfonyl, Phenylamino oder Phenylazo [welche gegebenenfalls durch Fluor, Chlor, Brom, Cyano, Nitro, Methyl und/oder Trifluormethyl substituiert sind], Pyridoxy oder Pyrimidoxy [welche gegebenenfalls durch Fluor, Chlor, Brom, Cyano, Nitro, Methyl und/oder Trifluormethyl substituiert sind], $C_1$-$C_4$-Alkyl-carbonyloxy, $C_1$-$C_4$-Alkoxy-carbonyloxy, $C_1$-$C_4$-Alkylamino-carbonyloxy und Di-($C_1$-$C_4$-alkyl)-aminocarbonyloxy, oder welcher gegebenenfalls durch eine Alkylenkette [welche gegebenenfalls verzweigt und/oder durch ein oder mehrere Sauerstoffatome unterbrochen ist] oder einen Benzorest [welcher gegebenenfalls durch Fluor, Chlor, Brom, Cyano, Nitro, Methyl und/oder Trifluormethyl substituiert ist] anelliert ist; worin weiter

$R^3$ für Wasserstoff, Fluor, Chlor, Brom, Hydroxy, $C_1$-$C_4$-Alkylamino, Di-($C_1$-$C_4$-alkyl)-amino, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist] steht und

$R^4$ für Wasserstoff, Fluor, Chlor, Brom, Hydroxy, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], $C_1$-$C_4$-Alkylthio [welches gegebenenfalls durch Fluor und/oder Chlor substituiert ist], $C_1$-$C_4$-Alkylamino oder Di-($C_1$-$C_4$-alkyl)-amino steht.

3) O-Aryl-N'-(triazin-2-yl)-isoharnstoffe der Formel (I) gemäß Anspruch 1, dadurch gekennzeichnet, daß darin

$R^1$ für den Rest

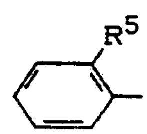

steht, worin

$R^5$ für Difluormethoxy, Trifluormethoxy, Difluormethylthio, Trifluormethylthio, Dimethylaminocarbonyl, Diethylaminocarbonyl, Di-n-propylaminocarbonyl, Methylsulfonyl, Ethylsulfonyl, n-Propylsulfonyl, i-Propylsulfonyl, Dimethylaminosulfonyl, Diethylaminosulfonyl, Methoxyaminosulfonyl, N-Methoxy-N-methylaminosulfonyl, Phenyl oder für Phenoxy, steht und

$R^2$ für einen Phenylrest steht, welche gegebenenfalls substituiert ist durch einen oder zwei Reste aus der Reihe

Fluor, Chlor, Brom, Iod, Cyano, Nitro, Hydroxy, Carboxy, $C_1$-$C_3$-Alkoxy-carbonyl, $C_1$-$C_4$-Alkyl, Trifluormethyl, Hydroxymethyl, Methoxycarbonylmethyl, Phenyl-$C_1$-$C_3$-alkyl, Cyclohexyl, $C_1$-$C_3$-Alkoxy, Trifluormethoxy, $C_1$-$C_3$-Alkylthio, Trifluormethylthio, Dimethylamino, Amino, Acetylamino, Methylaminocarbonyl, Formyl, Acetyl, Benzoyl, Phenyl, Hydroxyphenyl, Phenoxy [welches gegebenenfalls durch Chlor und/oder Trifluormethyl substituiert ist], Phenylamino, Phenylazo, Pydridoxy [welches gegebenenfalls durch Chlor und/oder Trifluormethyl substituiert ist], oder welcher gegebenenfalls benzanelliert ist, worin weiter

$R^3$ für Fluor, Chlor, Methyl, Ethyl, Methoxy, Ethoxy, Methylthio, Ethylthio, Methylamino, Ethylamino, Dimethylamino oder Diethylamino steht und

$R^4$ für Fluor, Chlor, Methyl, Ethyl, Methoxy, Ethoxy, Methylthio, Ethylthio, Methylamino, Ethylamino, Dimethylamino oder Diethylamino steht.

4) Verfahren zur Herstellung von O-Aryl-N'-(triazin-2-yl)-isoharnstoffen der Formel (I) gemäß Anspruch 1, dadurch gekennzeichnet, daß man Iminokohlensäureester der Formel (II)

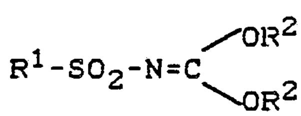

(II)

in welcher

$R^1$ und $R^2$ die oben in Anspruch 1 angegebenen Bedeutungen haben,

mit 2-Amino-triazinen der Formel (III)

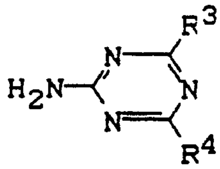

(III)

in welcher
$R^3$ und $R^4$ die oben in Anspruch 1 angegebenen Bedeutungen haben,
in Gegenwart von Säureakzeptoren und gegebenenfalls in Gegenwart von Verdünnungsmitteln umgesetzt.

5) Herbizide Mittel, gekennzeichnet durch ein Gehalt an mindestens einem O-Aryl-N'-(triazin-2-yl)-isoharnstoff der allgemeinen Formel (I) gemäß Anspruch 1.

6) Verwendung von O-Aryl-N'-(triazin-2-yl)-isoharnstoffen der allgemeinen Formel (I) gemäß Anspruch 1 zur Bekämpfung von unerwünschten Pflanzenwachstum.

7) Verfahren zur Herstellung von herbiziden Mitteln, dadurch gekennzeichnet, daß man O-Aryl-N'-(triazin-2-yl)isoharnstoffe der allgemeinen Formel (I) gemäß Anspruch 1 mit Streckmitteln und/oder oberflächenaktiven Mitteln vermischt.